# EUROPEAN PATENT APPLICATION

(11) **EP 4 324 912 A1**
(43) Date of publication of application: **21.02.2024**
(21) Application number: 22788077.0
(22) Date of filing: 31.03.2022
(51) Int. Cl.: C12N 5/04, A23L 27/00, A61K 47/02, A61K 47/18, A61K 47/26, A61K 47/46, A01H 5/02, A01H 5/06, A01H 5/10, A01H 5/12, C12Q 1/6869, C12Q 1/6895, A01H 6/00, A23L 2/38, A23L 33/105

(54) **STEVIA PLANT RICH IN NUTRITIONAL COMPONENT**

(30) Priority: 15.04.2021 JP 2021069151
(71) Applicant: Suntory Holdings Limited, Osaka 530-8203 (JP)
(72) Inventor: HIRAI, Tadayoshi, Kyoto 619-0284 (JP); IWAKI, Kazunari, Kawasaki-shi, Kanagawa 211-0067 (JP); OCHIAI, Kentaro, Kyoto 619-0284 (JP); TAKEYAMA, Saori, Kawasaki-shi, Kanagawa 211-0067 (JP); MIYAGAWA Katsuro, Kyoto 619-0284 (JP)
(74) Representative: Mewburn Ellis LLP
(86) International application number: PCT/JP2022/016463
(87) International publication number: WO 2022/220152

(57) **Abstract**

The present invention provides a stevia plant having a genotype C/C at a position corresponding to position 37 of SEQ ID NO: 1. The present invention also provides a method of producing the stevia plant, and an extract obtained from the plant.

## Description

### TECHNICAL FIELD

The present invention relates to a stevia plant having the genotype C/C at a position corresponding to position 37 of SEQ ID NO: 1, a method for producing the same and a method for screening for the same, etc.

### BACKGROUND ART

Stevia is a perennial plant of the family *Asteraceae* with Paraguay in the South America as its place of origin. Stevia contains a sweet component having several tens to several hundreds of times the sweetness of sugar, and such a sweet component is extracted therefrom and used as a natural sweetener (Patent Literature 1). However, much remains unknown about gene information or what kind of gene is involved in the control of *in vivo* events in stevia, for example.

### CITATION LIST

### PATENT LITERATURE

Patent Literature 1: WO2018/124142

### SUMMARY OF INVENTION

### TECHNICAL PROBLEM

It is desired to further elucidate gene information on stevia.

### MEANS FOR SOLVING THE PROBLEMS

The present invention provides a nutrient-rich stevia plant, a method of producing the plant, and a method of screening for the plant, etc.

In one embodiment, the present invention provides the following.
[1] A stevia plant having the genotype C/C at a position corresponding to position 37 of SEQ ID NO: 1.
[2] The plant according to [1], wherein the plant contains at least one nutrient component selected from the group consisting of iron, zinc, phosphorus, copper, molybdenum, amino acid, rebaudioside D, and rebaudioside M in a larger amount than a control stevia plant having a genotype C/T at a position corresponding to position 37 of SEQ ID NO: 1, and/or has an FRO2 expression level lower than in the control stevia plant.
[3] The plant according to [1] or [2], wherein the plant is a non-genetically modified plant.
[4] A seed, a tissue, a tissue culture or a cell of the plant according to any one of [1] to [3],
[5] The tissue, tissue culture or cell according to [4], which is selected from an embryo, a meristem cell, a pollen, a leaf, a root, a root apex, a petal, a protoplast, a leaf section and a callus.
[6] A method of producing a stevia plant containing at least one component selected from the group consisting of iron, zinc, phosphorus, copper, molybdenum, amino acid, rebaudioside D, and rebaudioside M in a larger amount than in a control stevia plant having a genotype C/T at a position corresponding to position 37 of SEQ ID NO: 1, and/or having an FRO2 expression level lower than in the control stevia plant, the method comprising a step of crossing the plant according to any one of [1] to [3] with a second stevia plant.
[7] The method according to [6], wherein the second plant is the plant according to any one of [1] to [3],
[8] An extract of the plant according to any one of [1] to [3], or of the seed, tissue, tissue culture or cell according to [4] or [5], wherein the extract comprises at least one nutrient component selected from iron, zinc, phosphorus, copper, molybdenum, amino acid, rebaudioside D, and rebaudioside M.
[9] A method of producing an extract comprises at least one nutrient component selected from iron, zinc, phosphorus, copper, molybdenum, amino acid, rebaudioside D, and rebaudioside M, wherein the method comprising a step of obtaining an extract from the plant according to any one of [1] to [3], or from the seed, tissue, tissue culture or cell according to [4] or [5].
[10] A method of producing a food or beverage, a sweetener composition, a flavor or a medicament, comprising a step of providing an extract of the plant according to any one of [1] to [3], an extract of the seed, tissue, tissue culture or cell according to [4] or [5], or the extract according to [8], and a step of adding the extract to a raw material for the food or beverage, sweetener composition, flavor or medicament.
[11] A herbal tea product comprising a dry matter of the plant according to any one of [1] to [3].
[12] A method of screening for the stevia plant according to any one of [1] to [3], the method comprising a step of detecting, in a test stevia plant, whether or not a genotype at a position corresponding to position 37 of SEQ ID NO: 1 is C/C.
[13] The method according to [12], wherein the step of detecting a genetic feature is performed by use of sequencing, dCAPS method or TaqMan PCR method.
[14] The method according to [12] or [13], further comprising a step of evaluating the content of at least one nutrient component selected from iron, zinc, phosphorus, copper, molybdenum, amino acid, rebaudioside D, and rebaudioside M in a test stevia plant tissue.
[15] A screening kit for the stevia plant according to any one of [1]-[3], comprising a reagent for detecting whether or not a genotype at a position corresponding to position 37 of SEQ ID NO: 1 is C/C.
[16] The kit according to [15], wherein the reagent comprises a primer and/or a probe for use in dCAPS method or TaqMan PCR method.
[17] A method of screening for a plant having a high content of at least one nutrient component selected from the group consisting of iron, zinc, phosphorus, copper, molybdenum, amino acid, rebaudioside D, and rebaudioside M, the method comprising a step of measuring an FRO2 expression level.

### ADVANTAGEOUS EFFECTS OF INVENTION

The present invention enables the obtainment of a nutrient-rich stevia plant and the provision of an approach for producing such a plant, a leaf obtainable from such a plant, and a food, a beverage, etc. containing an extract obtained from this leaf.

### BRIEF DESCRIPTION OF DRAWINGS

FIG. 1 is a diagram showing a variation site (position 37) in a nucleotide sequence of SEQ ID NO: 1. The base surrounded by a frame is a base (C or T) in the variation site.
FIG. 2 is a diagram showing RebD and RebM contents in each individual having a genotype C/C or C/T.
FIG. 3 is a diagram showing FRO2 gene expression level in individuals having the genotype C/C or C/T.
FIG. 4 is a graph showing a ratio of an average content of each mineral component in individuals having the genotype C/C obtained when an average content of each mineral component in individuals having the genotype C/T is defined as 1.
FIG. 5 is a graph showing a ratio of an average content of each amino acid in individuals having the genotype C/C obtained when an average content of each amino acid in individuals having the genotype C/T is defined as 1.
FIG. 6 is a graph showing a relationship between total content of RebD and RebM and FRO2 expression level.
FIG. 7 is a graph showing comparison in the total content of RebD and RebM between an individual group having an FRO2 expression level falling in bottom 15% and an individual group having an FRO2 expression level falling in top 15%.
FIG. 8 is a graph showing a relationship between a ratio of the total content of RebD and RebM to TSG content (RebDMlTSG) and FRO2 expression level.

### DESCRIPTION OF EMBODIMENTS

The present invention will now be described in detail. The following embodiments are provided for illustrating the present invention and are not intended to limit the present invention only thereto. The present invention may be implemented in various forms, without departing from the spirit of the present invention.

Note that all documents, as well as laid-open application publications, patent application publications, and other patent documents cited herein shall be incorporated herein by reference. The present specification incorporates the contents of the specification and the drawings of Japanese Patent Application No. 2021-069151, filed on April 15, 2021, from which the present application claims priority.

### 1. Stevia plant having genotype C/C at position corresponding to position 37 of SEQ ID NO: 1

The present invention provides a stevia plant having a genotype C/C at a position corresponding to position 37 of SEQ ID NO: 1 (i.e., homozygous for the allele wherein the base at a position corresponding to position 37 of SEQ ID NO: 1 is C) (hereinafter, referred to as the "plant of the present invention" or the "stevia plant of the present invention"). Hereinafter, a genetic feature of having the genotype C/C at a position corresponding to position 37 of SEQ ID NO: 1 is referred to as the "genetic feature of the present invention".

Stevia is a plant having a scientific name of *Stevia Rebaudiana Bertoni.*

The phrase "position corresponding to" means the following. In case a sequence identical to a reference sequence (i.e., SEQ ID NO: 1) is present in the genome, it means a position or a portion in the sequence (i.e., 37) present in the genome, and in case a sequence identical to the reference sequence is not present in the genome, it means a position or portion in a sequence in the genome corresponding to the reference sequence, which corresponds to the position in the reference sequence. Whether or not a sequence identical to or corresponding to the reference sequence exists in the genome can be determined by, for example, amplifying genomic DNA of the stevia plant of interest with a primer capable of amplifying the reference sequence by PCR, sequencing the amplified product, and performing alignment analysis between the obtained sequence and the reference sequence. Non-limiting examples of a sequence corresponding to a reference sequence include, for example, a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the reference sequence. The position corresponding to the position in the reference sequence in the sequence corresponding to the reference sequence in the genome can be determined by taking into account the nucleotide sequence before and after the position in the reference sequence and the like. For example, a position in the sequence corresponding to the reference sequence in the genome corresponding to a position in the reference sequence can be determined by an alignment analysis of a reference sequence with a sequence corresponding to a reference sequence in the genome.

For instance, in case the genome of a stevia plant has a portion consisting of a nucleotide sequence identical to SEQ ID NO: 1, "the position corresponding to position 37 of SEQ ID NO: 1" is position 37 from the 5' end of the portion consisting of a nucleotide sequence identical to SEQ ID NO: 1 in the genome. On the other hand, in case the genome of a stevia plant has a portion consisting of a nucleotide sequence which is not identical to, but which corresponds to SEQ ID NO: 1, the genome does not have a portion consisting of a nucleotide sequence identical to SEQ ID NO: 1. Therefore, "the position corresponding to position 37 of SEQ ID NO: 1" does not necessarily correspond to position 37 from the 5' end of the portion corresponding to SEQ ID NO: 1. However, it is possible to identify "the position corresponding to position 37 of SEQ ID NO: 1" in the genome of such a stevia plant by taking into account the nucleotide sequence before and after the position 37 of SEQ ID NO: 1, and the like. For instance, one can identify "the position corresponding to position 37 of SEQ ID NO: 1 " in the genome of a stevia plant by an alignment analysis of the nucleotide sequence of a portion corresponding to SEQ ID NO: 1" in the genome of a stevia plant and the nucleotide sequence of SEQ ID NO: 1.

"The portion consisting of a nucleotide sequence corresponding to SEQ ID NO: 1" means, for instance, a portion consisting of a nucleotide sequence having a sequence identity of 60% or more, 70% or more, 75% or more, 80% or more, 81% or more, 82% or more, 83% or more, 84% or more, 85% or more, 86% or more, 87% or more, 88% or more, 89% or more, 90% or more, 91% or more, 92% or more, 93% or more, 94% or more, 95% or more, 96% or more, 97% or more, 98% or more, 98.1% or more, 98.4% or more, 98.7% or more, 99% or more, 99.2% or more, 99.5% or more, or 99.8% or more to the nucleotide sequence of SEQ ID NO: 1.

In one embodiment, "the portion consisting of a nucleotide sequence corresponding to SEQ ID NO: 1" includes a portion of the genome of a stevia plant which can be amplified by PCR using a forward primer which hybridizes to a complementary sequence of a portion of 15 to 25 base long from the 5' end of SEQ ID NO: 1 and a reverse primer which hybridizes to a portion of 15 to 25 base long from the 3' end of SEQ ID NO: 1.

In a specific embodiment, "the portion consisting of a nucleotide sequence corresponding to SEQ ID NO: 1" includes, for instance, a portion of the genome of a stevia plant which can be amplified by PCR using a forward primer comprising the nucleotide sequence of SEQ ID NO: 2 and a reverse primer comprising the nucleotide sequence of SEQ ID NO: 3.

In a specific embodiment, "the allele wherein the base at a position corresponding to position 37 of SEQ ID NO: 1 is C" comprises the nucleotide sequence of SEQ ID NO: 4, 5, 6 or 20.

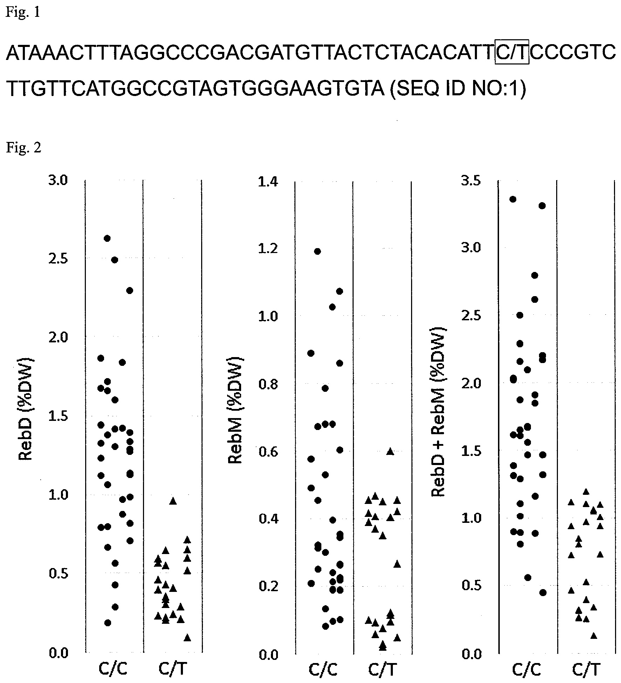

Here, the position corresponding to position 37 of SEQ ID NO: 1 may be referred to as a "polymorphic site of the present invention" or a "variation site of the present invention". Also, the variation from T to C at a position corresponding to position 37 of SEQ ID NO: 1 may be referred to as a "polymorphism of the present invention" or a "variation of the present invention". Fig. 1 indicates the position 37 of SEQ ID NO: 1.

The above genetic features can be detected by PCR method, TaqMan PCR method, sequencing method, microarray method, Invader method, TILLING method, RAD (random amplified polymorphic DNA) method, restriction fragment length polymorphism (RFLP) method, PCR-SSCP method, AFLP (amplified fragment length polymorphism) method, SSLP (simple sequence length polymorphism) method, CAPS (cleaved amplified polymorphic sequence) method, dCAPS (derived cleaved amplified polymorphic sequence) method, allele-specific oligonucleotide (ASO) method, ARMS method, denaturing gradient gel electrophoresis (DGGE) method, CCM (chemical cleavage of mismatch) method, DOL method, MALDI-TOF/MS method, TDI method, padlock probe method, molecular beacon method, DASH (dynamic allele specific hybridization) method, UCAN method, ECA method, PINPOINT method, PROBE (primer oligo base extension) method, VSET (very short extension) method, Survivor assay, Sniper assay, Luminex assay, GOOD method, LCx method, SNaPshot method, Mass ARRAY method, pyrosequencing method, SNP-IT method, melting curve analysis method, etc., but detection methods are not limited thereto.

In a specific embodiment, each genetic feature of the present invention is detectable using the following combination of a primer set and a restriction enzyme. In case where a candidate plant has the genetic feature of the present invention, for example, only a band of approximately 220 bp long (e.g., SEQ ID NO: 9) is obtained by: performing PCR amplification using a forward primer having the nucleotide sequence shown in SEQ ID NO: 7 and a reverse primer having the nucleotide sequence shown in SEQ ID NO: 8 on the genomic DNA of the candidate plant; and treating the obtained PCR product (approximately 220 bp long: e.g., SEQ ID NO: 9) with a AflIII restriction enzyme. On the other hand, when restriction enzyme-treated products of approximately 34 bp (e.g., SEQ ID NO: 11) and 186 bp (e.g., SEQ ID NO: 12) is formed by: obtaining, for example, a PCR product (approximately 220 bp long) of SEQ ID NO: 10 by PCR amplification; and treating the PCR product with a restriction enzyme AflIII, the candidate plant does not have the genetic feature of the present invention.

The term "approximately" as to bp long described above means ±5 bp. The restriction enzyme treatment can be performed according to conditions recommended by the distributor of each restriction enzyme used.

In one aspect, the plant of the present invention contains at least one nutrient component selected from the group consisting of minerals (e.g., iron, zinc, phosphorus, copper, molybdenum, etc.), amino acids (e.g., alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, etc.), rebaudioside D, and rebaudioside M in a larger amount than a control stevia plant having a genotype C/T at a position corresponding to position 37 of SEQ ID NO: 1 (hereinafter, referred to as the "chemical feature A of the present invention"). The phrase "containing in a larger amount than a control stevia plant" means, for example, that the content of the above component (e.g., the content in a dried leaf) is higher than that in the control stevia plant when the plants to be compared are cultivated under the same cultivation conditions. More specifically, the phrase means that the content of the component in a dried leaf is over 1-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.1-fold or more, 4.2-fold or more, 4.3-fold or more, 4.4-fold or more, 4.5-fold or more, 4.6-fold or more, 4.7-fold or more, 4.8-fold or more, 4.9-fold or more, 5.0-fold or more, 10-fold or more, 15-fold or more, 20-fold or more, 25-fold or more, 30-fold or more, 35-fold or more, 40-fold or more, 45-fold or more, 50-fold or more, 55-fold or more, 60-fold or more, 65-fold or more, 70-fold or more, 75-fold or more, 80-fold or more, 85-fold or more, 90-fold or more, 95-fold or more, or 100-fold or more of the content in the control stevia plant when the plants to be compared are cultivated, for example, under the same cultivation conditions. The content to be compared may be an average of those in a plurality of individuals. Also, the dried leaf refers to a leaf having a water content decreased to 3 to 4% by weight by drying a fresh leaf of the stevia plant of the present invention. Note that rebaudioside may be herein abbreviated as "Reb." or "Reb". For example, rebaudioside D may be abbreviated as "Reb.D" or "RebD".

In one embodiment, the plant of the present invention has a content of a mineral in a dried leaf higher than a content thereof in the control stevia plant when the plants to be compared are cultivated under the same cultivation conditions (hereinafter, referred to as the "chemical feature A1-a of the present invention"). The plant of the present invention may have a content of a mineral in a dried leaf of, for example, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.1-fold or more, 4.2-fold or more, 4.3-fold or more, 4.4-fold or more, 4.5-fold or more, 4.6-fold or more, 4.7-fold or more, 4.8-fold or more, 4.9-fold or more, 5.0-fold or more, 5.1-fold or more, 5.2-fold or more, 5.3-fold or more, 5.4-fold or more, 5.5-fold or more, 5.6-fold or more, 5.7-fold or more, 5.8-fold or more, 5.9-fold or more, 6.0-fold or more, 6.1-fold or more, 6.2-fold or more, 6.3-fold or more, 6.4-fold or more, 6.5-fold or more, 6.6-fold or more, 6.7-fold or more, 6.8-fold or more, 6.9-fold or more, or 7.0-fold or more of the content in the control stevia plant when the plants to be compared are cultivated under the same cultivation conditions.

In one embodiment, the plant of the present invention has a content of amino acid (free amino acid) in a dried leaf higher than a content thereof in the control stevia plant when the plants to be compared are cultivated under the same cultivation conditions (hereinafter, referred to as the "chemical feature A1-b of the present invention"). The plant of the present invention may have a content of amino acid in a dried leaf of, for example, over 1-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.1-fold or more, 4.2-fold or more, 4.3-fold or more, 4.4-fold or more, 4.5-fold or more, 5.0-fold or more, 10-fold or more, 15-fold or more, 20-fold or more, 25-fold or more, 30-fold or more, 35-fold or more, 40-fold or more, 45-fold or more, 50-fold or more, 55-fold or more, 60-fold or more, 65-fold or more, 70-fold or more, 75-fold or more, 80-fold or more, 85-fold or more, 90-fold or more, 95-fold or more, or 100-fold or more of the content in the control stevia plant when the plants to be compared are cultivated under the same cultivation conditions.

In a specific embodiment, the plant of the present invention has a content of iron in a dried leaf higher than a content thereof in the control stevia plant when the plants to be compared are cultivated under the same cultivation conditions (hereinafter, referred to as the "chemical feature A1-1 of the present invention"). The plant of the present invention may have a content of iron in a dried leaf of, for example, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, or 2.5-fold or more of the content in the control stevia plant when the plants to be compared are cultivated under the same cultivation conditions.

In a specific embodiment, the plant of the present invention has a content of zinc in a dried leaf higher than a content thereof in the control stevia plant when the plants to be compared are cultivated under the same cultivation conditions (hereinafter, referred to as the "chemical feature A1-2 of the present invention"). The plant of the present invention may have a content of zinc in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, or 2.0-fold or more of the content in the control stevia plant when the plants to be compared are cultivated under the same cultivation conditions.

In a specific embodiment, the plant of the present invention has a content of phosphorus (inorganic phosphorus) in a dried leaf higher than a content thereof in the control stevia plant when the plants to be compared are cultivated under the same cultivation conditions (hereinafter, referred to as the "chemical feature A1-3 of the present invention"). The plant of the present invention may have a content of phosphorus in a dried leaf of, for example, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, or 2.6-fold or more of the content in the control stevia plant when the plants to be compared are cultivated under the same cultivation conditions.

In a specific embodiment, the plant of the present invention has a content of copper in a dried leaf higher than a content thereof in the control stevia plant when the plants to be compared are cultivated under the same cultivation conditions (hereinafter, referred to as the "chemical feature A1-4 of the present invention"). The plant of the present invention may have a content of copper in a dried leaf of, for example, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.1-fold or more, 4.2-fold or more, 4.3-fold or more, 4.4-fold or more, 4.5-fold or more, 4.6-fold or more, 4.7-fold or more, 4.8-fold or more, 4.9-fold or more, or 5.0-fold or more of the content in the control stevia plant when the plants to be compared are cultivated under the same cultivation conditions.

In a specific embodiment, the plant of the present invention has a content of molybdenum in a dried leaf higher than a content thereof in the control stevia plant when the plants to be compared are cultivated under the same cultivation conditions (hereinafter, referred to as the "chemical feature A1-5 of the present invention"). The plant of the present invention may have a content of molybdenum in a dried leaf of, for example, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.1-fold or more, 4.2-fold or more, 4.3-fold or more, 4.4-fold or more, 4.5-fold or more, 4.6-fold or more, 4.7-fold or more, 4.8-fold or more, 4.9-fold or more, 5.0-fold or more, 5.1-fold or more, 5.2-fold or more, 5.3-fold or more, 5.4-fold or more, 5.5-fold or more, 5.6-fold or more, 5.7-fold or more, 5.8-fold or more, 5.9-fold or more, 6.0-fold or more, 6.1-fold or more, 6.2-fold or more, 6.3-fold or more, 6.4-fold or more, 6.5-fold or more, 6.6-fold or more, 6.7-fold or more, 6.8-fold or more, 6.9-fold or more, or 7.0-fold or more of the content in the control stevia plant when the plants to be compared are cultivated under the same cultivation conditions.

In a specific embodiment, the plant of the present invention has a content of alanine in a dried leaf higher than a content thereof in the control stevia plant when the plants to be compared are cultivated under the same cultivation conditions (hereinafter, referred to as the "chemical feature A1-6 of the present invention"). The plant of the present invention may have a content of alanine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more of the content in the control stevia plant when the plants to be compared are cultivated under the same cultivation conditions.

In a specific embodiment, the plant of the present invention has a content of arginine in a dried leaf higher than a content thereof in the control stevia plant when the plants to be compared are cultivated under the same cultivation conditions (hereinafter, referred to as the "chemical feature A1-7 of the present invention"). The plant of the present invention may have a content of arginine in a dried leaf of, for example, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 5.0-fold or more, 10-fold or more, 15-fold or more, 20-fold or more, 25-fold or more, 30-fold or more, 35-fold or more, 40-fold or more, 45-fold or more, 50-fold or more, 55-fold or more, 60-fold or more, 65-fold or more, 70-fold or more, 75-fold or more, 80-fold or more, 85-fold or more, 90-fold or more, 95-fold or more, or 100-fold or more of the content in the control stevia plant when the plants to be compared are cultivated under the same cultivation conditions.

In a specific embodiment, the plant of the present invention has a content of asparagine in a dried leaf higher than a content thereof in the control stevia plant when the plants to be compared are cultivated under the same cultivation conditions (hereinafter, referred to as the "chemical feature A1-8 of the present invention"). The plant of the present invention may have a content of asparagine in a dried leaf of, for example, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.8-fold or more, 4.0-fold or more, 4.3-fold or more, 4.5-fold or more, 4.8-fold or more, 5.0-fold or more, 5.3-fold or more, 5.5-fold or more, 5.8-fold or more, 6.0-fold or more, 6.3-fold or more, 6.5-fold or more, 6.8-fold or more, 7.0-fold or more, 7.3-fold or more, 7.5-fold or more, 7.8-fold or more, or 8.0-fold or more of the content in the control stevia plant when the plants to be compared are cultivated under the same cultivation conditions.

In a specific embodiment, the plant of the present invention has a content of aspartic acid in a dried leaf higher than a content thereof in the control stevia plant when the plants to be compared are cultivated under the same cultivation conditions (hereinafter, referred to as the "chemical feature A1-9 of the present invention"). The plant of the present invention may have a content of aspartic acid in a dried leaf of, for example, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 4.0-fold or more, 5.0-fold or more, 10-fold or more, 15-fold or more, 20-fold or more, 25-fold or more, 30-fold or more, 35-fold or more, 40-fold or more, 45-fold or more, 50-fold or more, 55-fold or more, 60-fold or more, 65-fold or more, 70-fold or more, 75-fold or more, 80-fold or more, 85-fold or more, 90-fold or more, 95-fold or more, or 100-fold or more of the content in the control stevia plant when the plants to be compared are cultivated under the same cultivation conditions.

In a specific embodiment, the plant of the present invention has a content of glutamic acid in a dried leaf higher than a content thereof in the control stevia plant when the plants to be compared are cultivated under the same cultivation conditions (hereinafter, referred to as the "chemical feature A1-10 of the present invention"). The plant of the present invention may have a content of glutamic acid in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 3.0-fold or more, 4.0-fold or more, 5.0-fold or more, 10-fold or more, 15-fold or more, 20-fold or more, 25-fold or more, 30-fold or more, 35-fold or more, 40-fold or more, 45-fold or more, 50-fold or more, 55-fold or more, 60-fold or more, 65-fold or more, 70-fold or more, 75-fold or more, 80-fold or more, 85-fold or more, 90-fold or more, 95-fold or more, or 100-fold or more of the content in the control stevia plant when the plants to be compared are cultivated under the same cultivation conditions.

In a specific embodiment, the plant of the present invention has a content of glycine in a dried leaf higher than a content thereof in the control stevia plant when the plants to be compared are cultivated under the same cultivation conditions (hereinafter, referred to as the "chemical feature A1-11 of the present invention"). The plant of the present invention may have a content of glycine in a dried leaf of, for example, 1.0-fold or more, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 3.0-fold or more, 4.0-fold or more, 5.0-fold or more, 10-fold or more, 15-fold or more, 20-fold or more, 25-fold or more, 30-fold or more, 35-fold or more, 40-fold or more, 45-fold or more, 50-fold or more, 55-fold or more, 60-fold or more, 65-fold or more, 70-fold or more, 75-fold or more, 80-fold or more, 85-fold or more, 90-fold or more, 95-fold or more, or 100-fold or more of the content in the control stevia plant when the plants to be compared are cultivated under the same cultivation conditions.

In a specific embodiment, the plant of the present invention has a content of histidine in a dried leaf higher than a content thereof in the control stevia plant when the plants to be compared are cultivated under the same cultivation conditions (hereinafter, referred to as the "chemical feature A1-12 of the present invention"). The plant of the present invention may have a content of histidine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, or 3.0-fold or more of the content in the control stevia plant when the plants to be compared are cultivated under the same cultivation conditions.

In a specific embodiment, the plant of the present invention has a content of isoleucine in a dried leaf higher than a content thereof in the control stevia plant when the plants to be compared are cultivated under the same cultivation conditions (hereinafter, referred to as the "chemical feature A1-13 of the present invention"). The plant of the present invention may have a content of isoleucine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, or 3.0-fold or more of the content in the control stevia plant when the plants to be compared are cultivated under the same cultivation conditions.

In a specific embodiment, the plant of the present invention has a content of leucine in a dried leaf higher than a content thereof in the control stevia plant when the plants to be compared are cultivated under the same cultivation conditions (hereinafter, referred to as the "chemical feature A1-14 of the present invention"). The plant of the present invention may have a content of leucine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, or 2.0-fold or more of the content in the control stevia plant when the plants to be compared are cultivated under the same cultivation conditions.

In a specific embodiment, the plant of the present invention has a content of lysine in a dried leaf higher than a content thereof in the control stevia plant when the plants to be compared are cultivated under the same cultivation conditions (hereinafter, referred to as the "chemical feature A1-15 of the present invention"). The plant of the present invention may have a content of lysine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, or 2.6-fold or more of the content in the control stevia plant when the plants to be compared are cultivated under the same cultivation conditions.

In a specific embodiment, the plant of the present invention has a content of phenylalanine in a dried leaf higher than a content thereof in the control stevia plant when the plants to be compared are cultivated under the same cultivation conditions (hereinafter, referred to as the "chemical feature A1-16 of the present invention"). The plant of the present invention may have a content of phenylalanine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, or 3.2-fold or more of the content in the control stevia plant when the plants to be compared are cultivated under the same cultivation conditions.

In a specific embodiment, the plant of the present invention has a content of proline in a dried leaf higher than a content thereof in the control stevia plant when the plants to be compared are cultivated under the same cultivation conditions (hereinafter, referred to as the "chemical feature A1-17 of the present invention"). The plant of the present invention may have a content of proline in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, or 2.7-fold or more of the content in the control stevia plant when the plants to be compared are cultivated under the same cultivation conditions.

In a specific embodiment, the plant of the present invention has a content of serine in a dried leaf higher than a content thereof in the control stevia plant when the plants to be compared are cultivated under the same cultivation conditions (hereinafter, referred to as the "chemical feature A1-18 of the present invention"). The plant of the present invention may have a content of serine in a dried leaf of, for example, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 5.0-fold or more, 6.0-fold or more, 7.0-fold or more, 8.0-fold or more, 9.0-fold or more, 10-fold or more, 11-fold or more, 12-fold or more, 13-fold or more, 14-fold or more, 15-fold or more, 16-fold or more, 17-fold or more, 18-fold or more, 19-fold or more, 20-fold or more, 21-fold or more, 22-fold or more, 23-fold or more, 24-fold or more, 25-fold or more, 26-fold or more, or 27-fold or more of the content in the control stevia plant when the plants to be compared are cultivated under the same cultivation conditions.

In a specific embodiment, the plant of the present invention has a content of threonine in a dried leaf higher than a content thereof in the control stevia plant when the plants to be compared are cultivated under the same cultivation conditions (hereinafter, referred to as the "chemical feature A1-19 of the present invention"). The plant of the present invention may have a content of threonine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, or 2.0-fold or more of the content in the control stevia plant when the plants to be compared are cultivated under the same cultivation conditions.

In a specific embodiment, the plant of the present invention has a content of tyrosine in a dried leaf higher than a content thereof in the control stevia plant when the plants to be compared are cultivated under the same cultivation conditions (hereinafter, referred to as the "chemical feature A1-20 of the present invention"). The plant of the present invention may have a content of tyrosine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, or 2.7-fold or more of the content in the control stevia plant when the plants to be compared are cultivated under the same cultivation conditions.

In a specific embodiment, the plant of the present invention has a content of valine in a dried leaf higher than a content thereof in the control stevia plant when the plants to be compared are cultivated under the same cultivation conditions (hereinafter, referred to as the "chemical feature A1-21 of the present invention"). The plant of the present invention may have a content of valine in a dried leaf of, for example, over 1.0-fold, 1.01-fold or more, 1.02-fold or more, 1.03-fold or more, 1.04-fold or more, 1.05-fold or more, 1.06-fold or more, 1.07-fold or more, 1.08-fold or more, 1.09-fold or more, 1.1-fold or more, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, or 2.2-fold or more of the content in the control stevia plant when the plants to be compared are cultivated under the same cultivation conditions.

In a specific embodiment, the plant of the present invention has a content of RebD in a dried leaf higher than a content thereof in the control stevia plant when the plants to be compared are cultivated under the same cultivation conditions (hereinafter, referred to as the "chemical feature A1-22 of the present invention"). The plant of the present invention may have a content of RebD in a dried leaf of, for example, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.1-fold or more, 4.2-fold or more, 4.3-fold or more, 4.4-fold or more, 4.5-fold or more, 5.0-fold or more, 6.0-fold or more, 7.0-fold or more, 8.0-fold or more, 9.0-fold or more, 10-fold or more, 11-fold or more, 12-fold or more, 13-fold or more, 14-fold or more, 15-fold or more, 16-fold or more, 17-fold or more, 18-fold or more, 19-fold or more, 20-fold or more, 21-fold or more, 22-fold or more, 23-fold or more, 24-fold or more, 25-fold or more, 26-fold or more, 27-fold or more, or 28-fold or more of the content in the control stevia plant when the plants to be compared are cultivated under the same cultivation conditions.

In a specific embodiment, the plant of the present invention has a content of RebM in a dried leaf higher than a content thereof in the control stevia plant when the plants to be compared are cultivated under the same cultivation conditions (hereinafter, referred to as the "chemical feature A1-23 of the present invention"). The plant of the present invention may have a content of RebM in a dried leaf of, for example, 1.2-fold or more, 1.3-fold or more, 1.4-fold or more, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 5.0-fold or more, 7.0-fold or more, 9.0-fold or more, 11-fold or more, 13-fold or more, 15-fold or more, 17-fold or more, 19-fold or more, 21-fold or more, 23-fold or more, 25-fold or more, 27-fold or more, 29-fold or more, 31-fold or more, 33-fold or more, 35-fold or more, 37-fold or more, 39-fold or more, 41-fold or more, 43-fold or more, 45-fold or more, 47-fold or more, 49-fold or more, 51-fold or more, or 53-fold or more of the content in the control stevia plant when the plants to be compared are cultivated under the same cultivation conditions.

In a specific embodiment, the plant of the present invention has a total content of RebD and RebM in a dried leaf higher than a content thereof in the control stevia plant when the plants to be compared are cultivated under the same cultivation conditions (hereinafter, referred to as the "chemical feature A1-24 of the present invention"). The plant of the present invention may have a total content of RebD and RebM in a dried leaf of, for example, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.5-fold or more, 5.0-fold or more, 6.0-fold or more, 7.0-fold or more, 8.0-fold or more, 9.0-fold or more, 10-fold or more, 11-fold or more, 12-fold or more, 13-fold or more, 14-fold or more, 15-fold or more, 16-fold or more, 17-fold or more, 18-fold or more, 19-fold or more, 20-fold or more, 21-fold or more, 22-fold or more, 23-fold or more, 24-fold or more, 25-fold or more, 26-fold or more, or 27-fold or more of the content in the control stevia plant when the plants to be compared are cultivated under the same cultivation conditions.

In a specific embodiment, the plant of the present invention has a weight ratio of RebD to Total Steviol Glycoside (RebD/TSG) in a dried leaf higher than a weight ratio thereof in the control stevia plant when the plants to be compared are cultivated under the same cultivation conditions (hereinafter, referred to as the "chemical feature A1-25 of the present invention"). The plant of the present invention may have RebD/TSG in a dried leaf of, for example, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.1-fold or more, 4.2-fold or more, 4.3-fold or more, 4.4-fold or more, 4.5-fold or more, 4.6-fold or more, 4.7-fold or more, 4.8-fold or more, 4.9-fold or more, 5.0-fold or more, 7.0-fold or more, 9.0-fold or more, 11-fold or more, 13-fold or more, 15-fold or more, 17-fold or more, 19-fold or more, 21-fold or more, 23-fold or more, 25-fold or more, 27-fold or more, 29-fold or more, 31-fold or more, 33-fold or more, 35-fold or more, 37-fold or more, 39-fold or more, 41-fold or more, 43-fold or more, or 45-fold or more of the content in the control stevia plant when the plants to be compared are cultivated under the same cultivation conditions.

TSG is a generic name for measurable steviol glycosides and includes neither an unknown steviol glycoside nor a steviol glycoside present at a level less than the detection limit. Preferably, TSG is any combination of two or more members selected from the group consisting of RebA, RebB, RebC, RebD, RebE, RebF, RebG, RebI, RebJ, RebK, RebM, RebN, RebO, RebQ, RebR, dulcoside A, rubusoside, steviolmonoside, steviolbioside and stevioside. In a specific embodiment, TSG consists of RebA, RebB, RebC, RebD, RebE, RebF, RebG, RebM, RebN and stevioside

In a specific embodiment, the plant of the present invention has a weight ratio of RebM to TSG (RebM/TSG) in a dried leaf higher than a weight ratio thereof in the control stevia plant when the plants to be compared are cultivated under the same cultivation conditions (hereinafter, referred to as the "chemical feature A1-26 of the present invention"). The plant of the present invention may have RebM/TSG in a dried leaf of, for example, 1.5-fold or more, 1.6-fold or more, 1.7-fold or more, 1.8-fold or more, 1.9-fold or more, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 4.0-fold or more, 4.5-fold or more, 5.0-fold or more, 10-fold or more, 15-fold or more, 20-fold or more, 25-fold or more, 30-fold or more, 35-fold or more, 40-fold or more, 45-fold or more, 50-fold or more, 55-fold or more, 60-fold or more, 65-fold or more, 70-fold or more, 75-fold or more, 80-fold or more, 85-fold or more, 90-fold or more, 95-fold or more, or 100-fold or more of the content in the control stevia plant when the plants to be compared are cultivated under the same cultivation conditions.

In a specific embodiment, the plant of the present invention has a weight ratio of the sum of RebD and RebM to TSG (RebDM/TSG) in a dried leaf higher than a weight ratio thereof in the control stevia plant when the plants to be compared are cultivated under the same cultivation conditions (hereinafter, referred to as the "chemical feature A1-27 of the present invention"). The plant of the present invention may have RebDM/TSG in a dried leaf of, for example, 2.0-fold or more, 2.1-fold or more, 2.2-fold or more, 2.3-fold or more, 2.4-fold or more, 2.5-fold or more, 2.6-fold or more, 2.7-fold or more, 2.8-fold or more, 2.9-fold or more, 3.0-fold or more, 3.1-fold or more, 3.2-fold or more, 3.3-fold or more, 3.4-fold or more, 3.5-fold or more, 3.6-fold or more, 3.7-fold or more, 3.8-fold or more, 3.9-fold or more, 4.0-fold or more, 4.1-fold or more, 4.2-fold or more, 4.3-fold or more, 4.4-fold or more, 4.5-fold or more, 4.6-fold or more, 4.7-fold or more, 4.8-fold or more, 4.9-fold or more, 5.0-fold or more, 6.0-fold or more, 8.0-fold or more, 10-fold or more, 12-fold or more, 14-fold or more, 16-fold or more, 18-fold or more, 20-fold or more, 22-fold or more, 24-fold or more, 26-fold or more, 28-fold or more, 30-fold or more, 32-fold or more, 34-fold or more, 36-fold or more, 38-fold or more, or 40-fold or more of the content in the control stevia plant when the plants to be compared are cultivated under the same cultivation conditions.

The plant of the present invention may have a combination of two or more of the chemical features described above. For example, the plant of the present invention may have the chemical features A1-a and A1-b, or may have at least two features out of the chemical features A1-1 to A1-27 (e.g., 2, 3, 4, 5, 6, 7, 8, 9, 10, 11, 12, 13, 14, 15, 16, 17, 18, 19, 20, 21, 22, 23, 24, 25, 26 or 27 features out of the chemical features A1-1 to A1-27), and may have, in a dried leaf, a mineral content 2.2-fold to 4.4-fold or more, a free amino acid content 1.1-fold to 3.8-fold or more, and a total content of RebD and RebM 2.4-fold or more as compared with the control stevia plant when the plants to be compared are cultivated, for example, under the same cultivation conditions. In one embodiment, the plant of the present invention has at least two features or more out of the following features (A1-1') to (A1-27'), and for example, has 2, 3, 4, 5, 6, 7, 8, 9, 10, 11, 12, 13, 14, 15, 16, 17, 18, 19, 20, 21, 22, 23, 24, 25, 26 or 27 features out of the features (A1-1') to (A1-27'):
(A1-1') iron content in a dried leaf is 2.2-fold or more as compared with that in the control stevia plant when cultivated under the same cultivation conditions;
(A1-2') zinc content in a dried leaf is 1.7-fold or more as compared with that in the control stevia plant when cultivated under the same cultivation conditions;
(A1-3') phosphorus content in a dried leaf is 2.2-fold or more as compared with that in the control stevia plant when cultivated under the same cultivation conditions;
(A1-4') copper content in a dried leaf is 4.2-fold or more as compared with that in the control stevia plant when cultivated under the same cultivation conditions;
(A1-5') molybdenum content in a dried leaf is 4.4-fold or more as compared with that in the control stevia plant when cultivated under the same cultivation conditions;
(A1-6') alanine content in a dried leaf is 1.2-fold or more as compared with that in the control stevia plant when cultivated under the same cultivation conditions;
(A1-7') arginine content in a dried leaf is 3.8-fold or more as compared with that in the control stevia plant when cultivated under the same cultivation conditions;
(A1-8') asparagine content in a dried leaf is 3.1-fold or more as compared with that in the control stevia plant when cultivated under the same cultivation conditions;
(A1-9') aspartic acid content in a dried leaf is 2.2-fold or more as compared with that in the control stevia plant when cultivated under the same cultivation conditions;
(A1-10') glutamine content in a dried leaf is 1.5-fold or more as compared with that in the control stevia plant when cultivated under the same cultivation conditions;
(A1-1 1') glycine content in a dried leaf is 1.5-fold or more as compared with that in the control stevia plant when cultivated under the same cultivation conditions;
(A1-12') histidine content in a dried leaf is 1.6-fold or more as compared with that in the control stevia plant when cultivated under the same cultivation conditions;
(A1-13') isoleucine content in a dried leaf is 1.5-fold or more as compared with that in the control stevia plant when cultivated under the same cultivation conditions;
(A1-14') leucine content in a dried leaf is 1.2-fold or more as compared with that in the control stevia plant when cultivated under the same cultivation conditions;
(A1-15') lysine content in a dried leaf is 1.4-fold or more as compared with that in the control stevia plant when cultivated under the same cultivation conditions;
(A1-16') phenylalanine content in a dried leaf is over 1.0-fold as compared with that in the control stevia plant when cultivated under the same cultivation conditions;
(A1-17') proline content in a dried leaf is 1.8-fold or more as compared with that in the control stevia plant when cultivated under the same cultivation conditions;
(A1-18') serine content in a dried leaf is 3.7-fold or more as compared with that in the control stevia plant when cultivated under the same cultivation conditions;
(A1-19') threonine content in a dried leaf is 1.5-fold or more as compared with that in the control stevia plant when cultivated under the same cultivation conditions;
(A1-20') tyrosine content in a dried leaf is 1.7-fold or more as compared with that in the control stevia plant when cultivated under the same cultivation conditions;
(A1-21') valine content in a dried leaf is 1.5-fold or more as compared with that in the control stevia plant when cultivated under the same cultivation conditions;
(A1-22') RebD content in a dried leaf is 2.9-fold or more as compared with that in the control stevia plant when cultivated under the same cultivation conditions;
(A1-23') RebM content in a dried leaf is 1.7-fold or more as compared with that in the control stevia plant when cultivated under the same cultivation conditions;
(A1-24') total content of RebD and RebM in a dried leaf is 2.4-fold or more as compared with that in the control stevia plant when cultivated under the same cultivation conditions;
(A1-25') weight ratio of RebD to TSG in a dried leaf is 3.8-fold or more as compared with that in the control stevia plant when cultivated under the same cultivation conditions;
(A1-26') weight ratio of RebM to TSG in a dried leaf is 2.1-fold or more as compared with that in the control stevia plant when cultivated under the same cultivation conditions; and
(A1-27') weight ratio of the sum of RebD and RebM to TSG in a dried leaf is 3.1-fold or more as compared with that in the control stevia plant when cultivated under the same cultivation conditions.

In a specific embodiment, the plant of the present invention may have a content of iron in a dried leaf of 3.0 ppb or more, 3.1 ppb or more, 3.2 ppb or more, 3.3 ppb or more, 3.4 ppb or more, 3.5 ppb or more, 3.6 ppb or more, 3.7 ppb or more, 3.8 ppb or more, 3.9 ppb or more, 4.0 ppb or more, 4.1 ppb or more, 4.2 ppb or more, 4.3 ppb or more, 4.4 ppb or more, 4.5 ppb or more, 4.6 ppb or more, 4.7 ppb or more, 4.8 ppb or more, 4.9 ppb or more, 5.0 ppb or more, 5.1 ppb or more, 5.2 ppb or more, 5.3 ppb or more, 5.4 ppb or more, 5.5 ppb or more, 5.6 ppb or more, 5.7 ppb or more, 5.8 ppb or more, 5.9 ppb or more, 6.0 ppb or more, 6.1 ppb or more, 6.2 ppb or more, 6.3 ppb or more, 6.4 ppb or more, 6.5 ppb or more, 6.6 ppb or more, 6.7 ppb or more, 6.8 ppb or more, 6.9 ppb or more, or 7.0 ppb or more (hereinafter, referred to as the "chemical feature A2-1 of the present invention").

In a specific embodiment, the plant of the present invention may have a content of zinc in a dried leaf of 55 ppb or more, 57 ppb or more, 60 ppb or more, 62 ppb or more, 65 ppb or more, 67 ppb or more, 70 ppb or more, 72 ppb or more, 75 ppb or more, 77 ppb or more, 80 ppb or more, 82 ppb or more, 85 ppb or more, 87 ppb or more, 90 ppb or more, 92 ppb or more, 95 ppb or more, 97 ppb or more, or 100 ppb or more(hereinafter, referred to as the "chemical feature A2-2 of the present invention").

In a specific embodiment, the plant of the present invention may have a content of phosphorus (inorganic phosphorus) in a dried leaf of 13000 ppb or more, 13500 ppb or more, 14000 ppb or more, 14500 ppb or more, 15000 ppb or more, 15500 ppb or more, 16000 ppb or more, 16500 ppb or more, 17000 ppb or more, 17500 ppb or more, 18000 ppb or more, 18500 ppb or more, 19000 ppb or more, 19500 ppb or more, 20000 ppb or more, 20500 ppb or more, 21000 ppb or more, 21500 ppb or more, 22000 ppb or more, 22500 ppb or more, 23000 ppb or more, 23500 ppb or more, 24000 ppb or more, 24500 ppb or more, 25000 ppb or more, 25500 ppb or more, 26000 ppb or more, 26500 ppb or more, 27000 ppb or more, 27500 ppb or more, 28000 ppb or more, 28500 ppb or more, 29000 ppb or more, 29500 ppb or more, 30000 ppb or more, 30500 ppb or more, 31000 ppb or more, 31500 ppb or more, 32000 ppb or more, 32500 ppb or more, 33000 ppb or more, 33500 ppb or more, 34000 ppb or more, 34500 ppb or more, or 35000 ppb or more (hereinafter, referred to as the "chemical feature A2-3 of the present invention").

In a specific embodiment, the plant of the present invention may have a content of copper in a dried leaf of 35 ppb or more, 40 ppb or more, 45 ppb or more, 50 ppb or more, 55 ppb or more, 60 ppb or more, 65 ppb or more, 70 ppb or more, 75 ppb or more, 80 ppb or more, 85 ppb or more, 90 ppb or more, 95 ppb or more, 100 ppb or more, 105 ppb or more, 110 ppb or more, 115 ppb or more, 120 ppb or more, 125 ppb or more, 130 ppb or more, 135 ppb or more, 140 ppb or more, 145 ppb or more, or 150 ppb or more (hereinafter, referred to as the "chemical feature A2-4 of the present invention").

In a specific embodiment, the plant of the present invention may have a content of molybdenum in a dried leaf of 0.30 ppb or more, 0.35 ppb or more, 0.40 ppb or more, 0.45 ppb or more, 0.50 ppb or more, 0.55 ppb or more, 0.60 ppb or more, 0.65 ppb or more, 0.70 ppb or more, 0.75 ppb or more, 0.80 ppb or more, 0.85 ppb or more, 0.90 ppb or more, 0.95 ppb or more, 1.00 ppb or more, 1.05 ppb or more, 1.10 ppb or more, 1.15 ppb or more, 1.20 ppb or more, 1.25 ppb or more, 1.30 ppb or more, 1.35 ppb or more, 1.40 ppb or more, 1.45 ppb or more, 1.50 ppb or more, 1.55 ppb or more, 1.60 ppb or more, 1.65 ppb or more, 1.70 ppb or more, 1.75 ppb or more, 1.80 ppb or more, 1.85 ppb or more, 1.90 ppb or more, 1.95 ppb or more, or 2.00 ppb or more (hereinafter, referred to as the "chemical feature A2-5 of the present invention").

In a specific embodiment, the plant of the present invention may have a content of alanine in a dried leaf of 21.0 ppm or more, 21.3 ppm or more, 21.5 ppm or more, 21.8 ppm or more, 22.0 ppm or more, 22.3 ppm or more, 22.5 ppm or more, 22.8 ppm or more, 23.0 ppm or more, 23.3 ppm or more, 23.5 ppm or more, 23.8 ppm or more, 24.0 ppm or more, 24.3 ppm or more, 24.5 ppm or more, 24.8 ppm or more, 25.0 ppm or more, 25.3 ppm or more, 25.5 ppm or more, 25.8 ppm or more, 26.0 ppm or more, 26.3 ppm or more, 26.5 ppm or more, 26.8 ppm or more, 27.0 ppm or more, 27.3 ppm or more, 27.5 ppm or more, 27.8 ppm or more, 28.0 ppm or more, 28.3 ppm or more, 28.5 ppm or more, 28.8 ppm or more, 29.0 ppm or more, 29.3 ppm or more, 29.5 ppm or more, 29.8 ppm or more, or 30.0 ppm or more (hereinafter, referred to as the "chemical feature A2-6 of the present invention").

In a specific embodiment, the plant of the present invention may have a content of arginine in a dried leaf of 1.0 ppm or more, 1.1 ppm or more, 1.2 ppm or more, 1.3 ppm or more, 1.4 ppm or more, 1.5 ppm or more, 1.6 ppm or more, 1.7 ppm or more, 1.8 ppm or more, 1.9 ppm or more, 2.0 ppm or more, 2.1 ppm or more, 2.2 ppm or more, 2.3 ppm or more, 2.4 ppm or more, 2.5 ppm or more, 2.6 ppm or more, 2.7 ppm or more, 2.8 ppm or more, 2.9 ppm or more, 3.0 ppm or more, 3.1 ppm or more, 3.2 ppm or more, 3.3 ppm or more, 3.4 ppm or more, 3.5 ppm or more, 3.6 ppm or more, 3.7 ppm or more, 3.8 ppm or more, 3.9 ppm or more, 4.0 ppm or more, 4.1 ppm or more, 4.2 ppm or more, 4.3 ppm or more, 4.4 ppm or more, or 4.5 ppm or more (hereinafter, referred to as the "chemical feature A2-7 of the present invention").

In a specific embodiment, the plant of the present invention may have a content of asparagine in a dried leaf of 2.5 ppm or more, 2.8 ppm or more, 3.0 ppm or more, 3.3 ppm or more, 3.5 ppm or more, 3.8 ppm or more, 4.0 ppm or more, 4.3 ppm or more, 4.5 ppm or more, 4.8 ppm or more, 5.0 ppm or more, 5.3 ppm or more, 5.5 ppm or more, 5.8 ppm or more, 6.0 ppm or more, 6.3 ppm or more, 6.5 ppm or more, 6.8 ppm or more, 7.0 ppm or more, 7.3 ppm or more, 7.5 ppm or more, 7.8 ppm or more, 8.0 ppm or more, 8.3 ppm or more, 8.5 ppm or more, 8.8 ppm or more, 9.0 ppm or more, 9.3 ppm or more, 9.5 ppm or more, 9.8 ppm or more, 10.0 ppm or more, 10.3 ppm or more, 10.5 ppm or more, 10.8 ppm or more, 11.0 ppm or more, 11.3 ppm or more, 11.5 ppm or more, 11.8 ppm or more, or 12.0 ppm or more (hereinafter, referred to as the "chemical feature A2-8 of the present invention").

In a specific embodiment, the plant of the present invention may have a content of aspartic acid in a dried leaf of 0.5 ppm or more, 0.6 ppm or more, 0.7 ppm or more, 0.8 ppm or more, 0.9 ppm or more, 1.0 ppm or more, 1.1 ppm or more, 1.2 ppm or more, 1.3 ppm or more, 1.4 ppm or more, or 1.5 ppm or more (hereinafter, referred to as the "chemical feature A2-9 of the present invention").

In a specific embodiment, the plant of the present invention may have a content of glutamic acid in a dried leaf of 1.5 ppm or more, 1.6 ppm or more, 1.7 ppm or more, 1.8 ppm or more, 1.9 ppm or more, 2.0 ppm or more, 2.1 ppm or more, 2.2 ppm or more, 2.3 ppm or more, 2.4 ppm or more, 2.5 ppm or more, 2.6 ppm or more, 2.7 ppm or more, 2.8 ppm or more, 2.9 ppm or more, or 3.0 ppm or more (hereinafter, referred to as the "chemical feature A2-10 of the present invention").

In a specific embodiment, the plant of the present invention may have a content of glycine in a dried leaf of 0.4 ppm or more, 0.5 ppm or more, 0.6 ppm or more, 0.7 ppm or more, 0.8 ppm or more, 0.9 ppm or more, or 1.0 ppm or more (hereinafter, referred to as the "chemical feature A2-11 of the present invention").

In a specific embodiment, the plant of the present invention may have a content of histidine in a dried leaf of 0.9 ppm or more, 1.0 ppm or more, 1.1 ppm or more, 1.2 ppm or more, 1.3 ppm or more, 1.4 ppm or more, or 1.5 ppm or more (hereinafter, referred to as the "chemical feature A2-12 of the present invention").

In a specific embodiment, the plant of the present invention may have a content of isoleucine in a dried leaf of 3.1 ppm or more, 3.2 ppm or more, 3.3 ppm or more, 3.4 ppm or more, 3.5 ppm or more, 3.6 ppm or more, 3.7 ppm or more, 3.8 ppm or more, 3.9 ppm or more, 4.0 ppm or more, 4.1 ppm or more, 4.2 ppm or more, 4.3 ppm or more, 4.4 ppm or more, 4.5 ppm or more, 4.6 ppm or more, 4.7 ppm or more, 4.8 ppm or more, 4.9 ppm or more, 5.0 ppm or more, 5.1 ppm or more, 5.2 ppm or more, 5.3 ppm or more, 5.4 ppm or more, or 5.5 ppm or more (hereinafter, referred to as the "chemical feature A2-13 of the present invention").

In a specific embodiment, the plant of the present invention may have a content of leucine in a dried leaf of 7.6 ppm or more, 7.7 ppm or more, 7.8 ppm or more, 7.9 ppm or more, 8.0 ppm or more, 8.1 ppm or more, 8.2 ppm or more, 8.3 ppm or more, 8.4 ppm or more, 8.5 ppm or more, 8.6 ppm or more, 8.7 ppm or more, 8.8 ppm or more, 8.9 ppm or more, 9.0 ppm or more, 9.1 ppm or more, 9.2 ppm or more, 9.3 ppm or more, 9.4 ppm or more, 9.5 ppm or more, 9.6 ppm or more, 9.7 ppm or more, 9.8 ppm or more, 9.9 ppm or more, 10.0 ppm or more, 10.1 ppm or more, 10.2 ppm or more, 10.3 ppm or more, 10.4 ppm or more, 10.5 ppm or more, 10.6 ppm or more, 10.7 ppm or more, 10.8 ppm or more, 10.9 ppm or more, 11.0 ppm or more (hereinafter, referred to as the "chemical feature A2-14 of the present invention").

In a specific embodiment, the plant of the present invention may have a content of lysine in a dried leaf of 2.9 ppm or more, 3.0 ppm or more, 3.1 ppm or more, 3.2 ppm or more, 3.3 ppm or more, 3.4 ppm or more, 3.5 ppm or more, 3.6 ppm or more, 3.7 ppm or more, 3.8 ppm or more, 3.9 ppm or more, 4.0 ppm or more, 4.1 ppm or more, 4.2 ppm or more, 4.3 ppm or more, 4.4 ppm or more, 4.5 ppm or more, 4.6 ppm or more, 4.7 ppm or more, 4.8 ppm or more, 4.9 ppm or more, or 5.0 ppm or more (hereinafter, referred to as the "chemical feature A2-15 of the present invention").

In a specific embodiment, the plant of the present invention may have a content of phenylalanine in a dried leaf of 11.4 ppm or more, 11.6 ppm or more, 11.8 ppm or more, 12.0 ppm or more, 12.2 ppm or more, 12.4 ppm or more, 12.6 ppm or more, 12.8 ppm or more, 13.0 ppm or more, 13.2 ppm or more, 13.4 ppm or more, 13.6 ppm or more, 13.8 ppm or more, 14.0 ppm or more, 14.2 ppm or more, 14.4 ppm or more, 14.6 ppm or more, 14.8 ppm or more, 15.0 ppm or more, 15.2 ppm or more, 15.4 ppm or more, 15.6 ppm or more, 15.8 ppm or more, or 16.0 ppm or more (hereinafter, referred to as the "chemical feature A2-16 of the present invention").

In a specific embodiment, the plant of the present invention may have a content of proline in a dried leaf of 2.8 ppm or more, 2.9 ppm or more, 3.0 ppm or more, 3.1 ppm or more, 3.2 ppm or more, 3.3 ppm or more, 3.4 ppm or more, 3.5 ppm or more, 3.6 ppm or more, 3.7 ppm or more, 3.8 ppm or more, 3.9 ppm or more, 4.0 ppm or more, 4.1 ppm or more, 4.2 ppm or more, 4.3 ppm or more, 4.4 ppm or more, 4.5 ppm or more, 4.6 ppm or more, 4.7 ppm or more, 4.8 ppm or more, 4.9 ppm or more, 5.0 ppm or more, 5.1 ppm or more, 5.2 ppm or more, 5.3 ppm or more, 5.4 ppm or more, 5.5 ppm or more, 5.6 ppm or more, 5.7 ppm or more, 5.8 ppm or more, 5.9 ppm or more, or 6.0 ppm or more (hereinafter, referred to as the "chemical feature A2-17 of the present invention").

In a specific embodiment, the plant of the present invention may have a content of serine in a dried leaf of 27 ppm or more, 30 ppm or more, 32 ppm or more, 35 ppm or more, 37 ppm or more, 40 ppm or more, 42 ppm or more, 45 ppm or more, 47 ppm or more, 50 ppm or more, 52 ppm or more, 55 ppm or more, 57 ppm or more, 60 ppm or more, 62 ppm or more, 65 ppm or more, 67 ppm or more, 70 ppm or more, 72 ppm or more, 75 ppm or more, 77 ppm or more, 80 ppm or more, 82 ppm or more, 85 ppm or more, 87 ppm or more, 90 ppm or more, 92 ppm or more, 95 ppm or more, 97 ppm or more, 100 ppm or more, 102 ppm or more, 105 ppm or more, 107 ppm or more, 110 ppm or more, 112 ppm or more, 115 ppm or more, 117 ppm or more, 120 ppm or more, 122 ppm or more, 125 ppm or more, 127 ppm or more, 130 ppm or more, 132 ppm or more, 135 ppm or more, 137 ppm or more, 140 ppm or more, 142 ppm or more, 145 ppm or more, 147 ppm or more, 150 ppm or more, 152 ppm or more, 155 ppm or more, 157 ppm or more, or 160 ppm or more (hereinafter, referred to as the "chemical feature A2-18 of the present invention").

In a specific embodiment, the plant of the present invention may have a content of threonine in a dried leaf of 3.8 ppm or more, 3.9 ppm or more, 4.0 ppm or more, 4.1 ppm or more, 4.2 ppm or more, 4.3 ppm or more, 4.4 ppm or more, 4.5 ppm or more, 4.6 ppm or more, 4.7 ppm or more, 4.8 ppm or more, 4.9 ppm or more, 5.0 ppm or more, 5.1 ppm or more, 5.2 ppm or more, 5.3 ppm or more, 5.4 ppm or more, or 5.5 ppm or more (hereinafter, referred to as the "chemical feature A2-19 of the present invention").

In a specific embodiment, the plant of the present invention may have a content of tyrosine in a dried leaf of 2.2 ppm or more, 2.3 ppm or more, 2.4 ppm or more, 2.5 ppm or more, 2.6 ppm or more, 2.7 ppm or more, 2.8 ppm or more, 2.9 ppm or more, 3.0 ppm or more, 3.1 ppm or more, 3.2 ppm or more, 3.3 ppm or more, 3.4 ppm or more, or 3.5 ppm or more (hereinafter, referred to as the "chemical feature A2-20 of the present invention").

In a specific embodiment, the plant of the present invention may have a content of valine in a dried leaf of 3.7 ppm or more, 3.8 ppm or more, 3.9 ppm or more, 4.0 ppm or more, 4.1 ppm or more, 4.2 ppm or more, 4.3 ppm or more, 4.4 ppm or more, 4.5 ppm or more, 4.6 ppm or more, 4.7 ppm or more, 4.8 ppm or more, 4.9 ppm or more, 5.0 ppm or more, 5.1 ppm or more, 5.2 ppm or more, 5.3 ppm or more, 5.4 ppm or more, 5.5 ppm or more, 5.6 ppm or more, 5.7 ppm or more, or 5.8 ppm or more (hereinafter, referred to as the "chemical feature A2-21 of the present invention").

In a specific embodiment, the plant of the present invention may have a content of RebD in a dried leaf of 0.5% by weight or more, 0.6% by weight or more, 0.7% by weight or more, 0.8% by weight or more, 0.9% by weight or more, 1.0% by weight or more, 1.1% by weight or more, 1.2% by weight or more, 1.3% by weight or more, 1.4% by weight or more, 1.5% by weight or more, 1.6% by weight or more, 1.7% by weight or more, 1.8% by weight or more, 1.9% by weight or more, 2.0% by weight or more, 2.1% by weight or more, 2.2% by weight or more, 2.3% by weight or more, 2.4% by weight or more, 2.5% by weight or more, 2.6% by weight or more, 2.7% by weight or more, 2.8% by weight or more, 2.9% by weight or more, 3.0% by weight or more, 3.1% by weight or more, 3.2% by weight or more, 3.3% by weight or more, 3.4% by weight or more, or 3.5% by weight or more (hereinafter, referred to as the "chemical feature A2-22 of the present invention").

In a specific embodiment, the plant of the present invention may have a content of RebM in a dried leaf of 0.27% by weight or more, 0.30% by weight or more, 0.35% by weight or more, 0.40% by weight or more, 0.45% by weight or more, 0.50% by weight or more, 0.55% by weight or more, 0.60% by weight or more, 0.65% by weight or more, 0.70% by weight or more, 0.75% by weight or more, 0.80% by weight or more, 0.85% by weight or more, 0.90% by weight or more, 0.95% by weight or more, 1.00% by weight or more, 1.05% by weight or more, 1.10% by weight or more, 1.15% by weight or more, 1.20% by weight or more, 1.25% by weight or more, 1.30% by weight or more, 1.35% by weight or more, 1.40% by weight or more, 1.45% by weight or more, or 1.50% by weight or more (hereinafter, referred to as the "chemical feature A2-23 of the present invention").

In a specific embodiment, the plant of the present invention may have a total content of RebD and RebM in a dried leaf of 0.8% by weight or more, 0.9% by weight or more, 1.0% by weight or more, 1.1% by weight or more, 1.2% by weight or more, 1.3% by weight or more, 1.4% by weight or more, 1.5% by weight or more, 1.6% by weight or more, 1.7% by weight or more, 1.8% by weight or more, 1.9% by weight or more, 2.0% by weight or more, 2.1% by weight or more, 2.2% by weight or more, 2.3% by weight or more, 2.4% by weight or more, 2.5% by weight or more, 2.6% by weight or more, 2.7% by weight or more, 2.8% by weight or more, 2.9% by weight or more, 3.0% by weight or more, 3.1% by weight or more, 3.2% by weight or more, 3.3% by weight or more, 3.4% by weight or more, 3.5% by weight or more, 3.6% by weight or more, 3.7% by weight or more, 3.8% by weight or more, 3.9% by weight or more, or 4.0% by weight or more (hereinafter, referred to as the "chemical feature A2-24 of the present invention").

In a specific embodiment, the plant of the present invention may have a weight ratio of RebD to TSG (RebD/TSG) in a dried leaf of 5% or more, 6% or more, 7% or more, 8% or more, 9% or more, 10% or more, 11% or more, 12% or more, 13% or more, 14% or more, 15% or more, 16% or more, 17% or more, 18% or more, 19% or more, 20% or more, 21% or more, 22% or more, 23% or more, 24% or more, 25% or more, 26% or more, 27% or more, 28% or more, 29% or more, or 30% or more (hereinafter, referred to as the "chemical feature A2-25 of the present invention").

In a specific embodiment, the plant of the present invention may have a weight ratio of RebM to TSG (RebM/TSG) in a dried leaf of 1.0% or more, 1.5% or more, 2.0% or more, 2.5% or more, 3.0% or more, 3.5% or more, 4.0% or more, 4.5% or more, 5.0% or more, 5.5% or more, 6.0% or more, 6.5% or more, 7.0% or more, 7.5% or more, 8.0% or more, 8.5% or more, 9.0% or more, 9.5% or more, 10.0% or more, 10.5% or more, 11.0% or more, 11.5% or more, 12.0% or more, 12.5% or more, 13.0% or more, 13.5% or more, 14.0% or more, 14.5% or more, 15.0% or more, 15.5% or more, 16.0% or more, 16.5% or more, 17.0% or more, 17.5% or more, or 18.0% or more (hereinafter, referred to as the "chemical feature A2-26 of the present invention").

In a specific embodiment, the plant of the present invention may have a weight ratio of the sum of RebD and RebM to TSG (RebDM/TSG) in a dried leaf of 5.5% or more, 7.0% or more, 8.5% or more, 10.0% or more, 11.5% or more, 13.0% or more, 14.5% or more, 16.0% or more, 17.5% or more, 19.0% or more, 20.5% or more, 22.0% or more, 23.5% or more, 25.0% or more, 26.5% or more, 28.0% or more, 29.5% or more, 31.0% or more, 32.5% or more, 34.0% or more, 35.5% or more, 37.0% or more, 38.5% or more, or 40.0% or more (hereinafter, referred to as the "chemical feature A2-27 of the present invention").

The plant of the present invention may have a combination of two or more of the features described above. For example, the plant of the present invention may have at least two features out of the chemical features A2-1 to A2-27 (e.g., 2, 3, 4, 5, 6, 7, 8, 9, 10, 11, 12, 13, 14, 15, 16, 17, 18, 19, 20, 21, 22, 23, 24, 25, 26 or 27 features out of the chemical features A2-1 to A2-27). In one embodiment, the plant of the present invention has at least two features or more out of the following features (A2-1') to (A2-27'), and for example, has 2, 3, 4, 5, 6, 7, 8, 9, 10, 11, 12, 13, 14, 15, 16, 17, 18, 19, 20, 21, 22, 23, 24, 25, 26 or 27 features out of the features (A2-1') to (A2-27'):
(A2-1') iron content in a dried leaf is 3.0 ppb or more;
(A2-2') zinc content in a dried leaf is 55 ppb or more;
(A2-3') phosphorus content in a dried leaf is 13000 ppb or more;
(A2-4') copper content in a dried leaf is 35 ppb or more;
(A2-5') molybdenum content in a dried leaf is 0.30 ppb or more;
(A2-6') alanine content in a dried leaf is 21.0 ppm or more;
(A2-7') arginine content in a dried leaf is1.0 ppm or more;
(A2-8') asparagine content in a dried leaf is 2.5 ppm or more;
(A2-9') aspartic acid content in a dried leaf is 0.5 ppm or more;
(A2-10') glutamine content in a dried leaf is 1.5 ppm or more;
(A2-1 1') glycine content in a dried leaf is 0.4 ppm or more;
(A2-12') histidine content in a dried leaf is 0.9 ppm or more;
(A2-13') isoleucine content in a dried leaf is 3.1 ppm or more;
(A2-14') leucine content in a dried leaf is 7.6 ppm or more;
(A2-15') lysine content in a dried leaf is 2.9 ppm or more;
(A2-16') phenylalanine content in a dried leaf is 11.4 ppm or more;
(A2-17') proline content in a dried leaf is 2.8 ppm or more;
(A2-18') serine content in a dried leaf is 27 ppm or more;
(A2-19') threonine content in a dried leaf is 3.8 ppm or more;
(A2-20') tyrosine content in a dried leaf is 2.2 ppm or more;
(A2-21') valine content in a dried leaf is 3.7 ppm or more;
(A2-22') RebD content in a dried leaf is 1.3% by weight or more;
(A2-23') RebM content in a dried leaf is 0.43% by weight or more;
(A2-24') total content of RebD and RebM in a dried leaf is 0.8% by weight or more;
(A2-25') weight ratio of RebD to TSG in a dried leaf is 18.8% or more;
(A2-26') weight ratio of RebM to TSG in a dried leaf is 6.6% or more; and
(A2-27') weight ratio of the sum of RebD and RebM to TSG in a dried leaf is 25.4% or more.

The mineral content can be measured by any known method such as ICP mass spectrometry described in Examples mentioned later, atomic absorption spectrometry, or ICP emission spectrometry. The amino acid content can be measured by any known method such as HPLC described in Examples mentioned later, precolumn derivatization, or postcolumn derivatization. The content of a steviol glycoside such as RebD or RebM can be measured by a method described in Ohta et al., J. Appl. Glycosci., Vol. 57, No. 3, 199-209 (2010) or WO2010/038911, or a method described in Examples mentioned later. More specifically, the content of a steviol glycoside can be measured by sampling a fresh leaf from the stevia plant, followed by measurement by LC/MS-MS or the like.

In another aspect, the plant of the present invention has a lower FRO2 expression level than the control stevia plant having the genotype C/T at a position corresponding to position 37 of SEQ ID NO: 1 (hereinafter, referred to as the "chemical feature B of the present invention"). The phrase "having a lower FRO2 expression level than the control stevia plant" means that the FRO2 expression level is lower than that in the control stevia plant when the plants to be compared are cultivated, for example, under the same cultivation conditions. More specifically, the phrase means that the expression level of FRO2 gene (e.g., FPKM value) in a fresh leaf is lower than the expression level of FRO2 gene in a fresh leaf of the control stevia plant, by 5.0% or more, 5.2% or more, 5.4% or more, 5.6% or more, 5.8% or more, 6.0% or more, 6.2% or more, 6.4% or more, 6.6% or more, 6.8% or more, 7.0% or more, 7.2% or more, 7.4% or more, 7.6% or more, 7.8% or more, 8.0% or more, 8.2% or more, 8.4% or more, 8.6% or more, 8.8% or more, 9.0% or more, 9.2% or more, 9.4% or more, 9.6% or more, 9.8% or more, or 10.0% or more when the plants to be compared are cultivated, for example, under the same cultivation conditions. The expression level to be compared may be a median of those of a plurality of individuals. In one embodiment, the plant of the present invention has the expression level of FRO2 gene in a fresh leaf lower than the expression level of FRO2 gene in a fresh leaf of the control stevia plant by 6.8% or more in terms of FPKM value when the plants to be compared are cultivated, for example, under the same cultivation conditions.

The FRO2 expression level can be measured by sequencing by NGS or the like described in Examples mentioned later, or any known method for measuring a gene expression level, such as various hybridization methods utilizing a nucleic acid molecule encoding FRO2 or a nucleic acid molecule specifically hybridizing its unique fragment (e.g., in situ hybridization), Northern blotting, Southern blotting, various PCRs, immunoprecipitation using a substance, such as an antibody, capable of specifically recognizing FRO2, EIA (e.g., ELISA, etc.), RIA (e.g., IRMA, RAST, RIST, etc.), Western blotting, immunohistochemistry, immunocytochemistry, flow cytometry, or MRI. The genomic sequence, the CDS sequence, and the amino acid sequence of stevia FRO2 are respectively shown in SEQ ID NOS: 13, 14, and 15.

In another aspect, the plant of the present invention contains RebA in a smaller amount than the control stevia plant having the genotype C/T at a position corresponding to position 37 of SEQ ID NO: 1 (hereinafter, referred to as the "chemical feature C1 of the present invention"). The phrase "containing RebA in a smaller amount than the control stevia plant" means that the RebA content (e.g., the content in a dried leaf) is lower than that in the control stevia plant when the plants to be compared are cultivated, for example, under the same cultivation conditions. More specifically, the plant of the present invention may have a RebA content 0.70 times or less, 0.68 times or less, 0.66 times or less, 0.64 times or less, 0.62 times or less, 0.60 times or less, 0.58 times or less, 0.56 times or less, 0.54 times or less, 0.52 times or less, 0.50 times or less, 0.48 times or less, 0.46 times or less, 0.44 times or less, 0.42 times or less, 0.40 times or less, 0.38 times or less, 0.36 times or less, 0.34 times or less, 0.32 times or less, 0.30 times or less, 0.28 times or less, 0.26 times or less, 0.24 times or less, 0.22 times or less, 0.20 times or less, 0.18 times or less, 0.16 times or less, 0.14 times or less, 0.12 times or less, or 0.10 times or less, in particular 0.48 times or less of the content in a fresh leaf of the control stevia plant when the plants to be compared are cultivated, for example, under the same cultivation conditions. The content to be compared may be an average of those of a plurality of individuals.

In a specific embodiment, the plant of the present invention may have a RebA content in a dried leaf of 10.0% by weight or less, 9.6% by weight or less, 9.2% by weight or less, 8.8% by weight or less, 8.4% by weight or less, 8.0% by weight or less, 7.6% by weight or less, 7.2% by weight or less, 6.8% by weight or less, 6.4% by weight or less, 6.0% by weight or less, 5.6% by weight or less, 5.2% by weight or less, 4.8% by weight or less, 4.4% by weight or less, 4.0% by weight or less, 3.6% by weight or less, 3.2% by weight or less, 2.8% by weight or less, 2.4% by weight or less, or 2.0% by weight or less (hereinafter referred to as the "chemical feature C2 of the present invention") when the plants are cultivated under the same cultivation conditions (hereinafter, referred to as the "chemical feature C2 of the present invention").

The plant of the present invention may have a combination of two or more of the chemical features described above. The plant of the present invention may have, for example, 2 or 3 features out of the chemical features A to C of the present invention. In a more specific embodiment, the plant of the present invention may have at least two features out of the chemical features A1-1 to A1-27, A2-1 to A2-27, B, C1, and C2 of the present invention, and may have, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58 or 59 features out of these chemical features.

The plant of the present invention may include not only the whole plant but a plant organ (e.g., a leaf, a petal, a stem, a root, and a seed), a plant tissue (e.g., epidermis, phloem, soft tissue, xylem, vascular bundle, palisade tissue, and spongy tissue), various forms of plant cells (e.g., suspended cultured cells), a protoplast, a leaf section, a callus, and the like. The leaf may be the dried leaf mentioned above.

The plant of the present invention may also include a tissue culture or a cultured plant cell. This is because the plant can be regenerated by culturing such a tissue culture or a cultured plant cell. Examples of the tissue culture or the cultured plant cell of the plant of the present invention include, but are not limited to, embryos, meristem cells, pollens, leaves, roots, root apices, petals, protoplasts, leaf sections and calluses.

### 2. Method of producing plant of present invention

In another aspect, the present invention provides a method of producing a stevia plant containing at least one component selected from the group consisting of iron, zinc, phosphorus, copper, molybdenum, amino acid, RebD, and RebM in a larger amount than a control stevia plant having the genotype C/T at a position corresponding to position 37 of SEQ ID NO: 1, and/or having an FRO2 expression level lower than in the control stevia plant, the method comprising a step of crossing the stevia plant of the present invention with a second stevia plant (hereinafter, referred to as the "production method of the present invention").

The stevia plant produced by the method may have the same genetic features as the plant of the present invention.

The ranges of the contents of minerals, amino acids, RebD and RebM, or the range of the weight ratio of RebD and RebM to TSG in the plant obtained by the production method of the present invention, the ranges of the contents or the ratio to the weight ratio in the control stevia plant, and the range of the ratio of the FRO2 expression level to the expression level in the control stevia plant are as described above about the plant of the present invention.

In the production method of the present invention, "hybridizing" means that the plant of the present invention (first generation (S1)) is crossed with a second plant (S1) to obtain a progeny plant thereof (plant produced by the production method of the present invention (second generation (S2)). The hybridizing method is preferably backcross. The "backcross" is an approach of further crossing a progeny plant (S2) generated between the plant of the present invention and the second plant, with the plant of the present invention (i.e., a plant having the genetic feature of the present invention) (S1) to produce a plant having the genetic feature of the present invention. When the second plant (S1) for use in the production method of the present invention has the same genetic feature as the one of the plant of the present invention, the crossing is substantially backcross.

Alternatively, the plant of the present invention can also be produced by selfing. The selfing can be performed by the self-pollination of the stamen pollen of the plant of the present invention with the pistil of the plant of the present invention.

Since the plant produced by the production method of the present invention has the same genetic feature as the one of the plant of the present invention, the plant produced by the production method of the present invention can be further crossed with a third stevia plant to produce a stevia plant having a phenotype equivalent to that of the plant of the present invention.

In an alternative embodiment, the plant of the present invention may be produced by regenerating a plant by the culture of the tissue culture or the cultured plant cell mentioned above. The culture conditions are the same as those for culturing a tissue culture or a cultured plant cell of the wild type stevia plant and are known in the art (Protocols for In Vitro cultures and secondary metabolite analysis of aromatic and medicinal plants, Method in molecular biology, vol. 1391, pp. 113-123).

In a further alternative embodiment, the plant of the present invention may be produced by introducing the variation of the present invention to the genome of a stevia plant. The introduction of the variation may be performed by a genetic modification approach or may be performed by a non-genetic modification approach. Examples of the "non-genetic modification approach" include a method of inducing a variation in the gene of a host cell (or a host plant) without transfection with a foreign gene. Examples of such a method include a method of allowing a mutagen to act on a plant cell. Examples of such a mutagen include ethylmethanesulfonate (EMS) and sodium azide. For example, EMS can be used at a concentration such as 0.1%, 0.2%, 0.3%, 0.4%, 0.5%, 0.6%, 0.7%, 0.8%, 0.9% or 1.0% to treat a plant cell. The treatment time is 1 to 48 hours, 2 to 36 hours, 3 to 30 hours, 4 to 28 hours, 5 to 26 hours, or 6 to 24 hours. The procedures themselves of the treatment are known in the art and can be performed by dipping a water-absorbed seed obtained through a water absorption process in a treatment solution containing the mutagen at the concentration described above for the treatment time described above.

An alternative example of the non-genetic modification approach can be a method of irradiating a plant cell with radiation or light beam such as X ray, γ ray, or ultraviolet ray. In this case, a cell irradiated using an appropriate dose (ultraviolet lamp intensity, distance, and time) of ultraviolet ray is cultured in a selective medium or the like, and then, a cell, a callus, or a plant having the trait of interest can be selected. In this operation, the irradiation intensity is 0.01 to 100 Gr, 0.03 to 75 Gr, 0.05 to 50 Gr, 0.07 to 25 Gr, 0.09 to 20 Gr, 0.1 to 15 Gr, 0.1 to 10 Gr, 0.5 to 10 Gr, or 1 to 10 Gr. The irradiation distance is 1 cm to 200 m, 5 cm to 100 m, 7 cm to 75 m, 9 cm to 50 m, 10 cm to 30 m, 10 cm to 20 m, 10 cm to 10 m. The irradiation time is 1 minute to 2 years, 2 minutes to 1 year, 3 minutes to 0.5 years, 4 minutes to 1 month, 5 minutes to 2 weeks, or 10 minutes to 1 week. The irradiation intensity, distance and time differ depending on the type of radiation or the state of the subject to be irradiated (cell, callus, or plant) and can be appropriately adjusted by those skilled in the art.

Approaches such as cell fusion, another culture (haploid induction), and remote crossing (haploid induction) are also known in the art.

In general, plant cells may involve a mutation during culture. Therefore, it is preferred to regenerate a plant individual, for more stably maintaining the trait.

The scope of the present invention does not exclude a plant obtained by the ex-post facto genetic recombination (e.g., genome editing) with the plant of the present invention as a host (e.g., a plant further provided with another trait by genetic recombination with the plant of the present invention as a host).

The plant of the present invention may be a plant obtained by a genetic modification approach or a progeny plant thereof (hereinafter, referred to as the "genetically modified plant"), or may be a plant obtained by a non-genetic modification approach or a progeny plant thereof (hereinafter, referred to as the "non-genetically modified plant").

### 3. Method of screening for plant of present invention

The plant of the present invention or the plant having the same genetic feature as the one of the plant of the present invention can be screened for by detecting the genetic feature of the present invention from a tissue of a test plant. In this context, "screening" means that the plant of the present invention is discriminated from the other plants to select the plant of the present invention.

Thus, in an alternative aspect, the present invention provides a method of screening for a stevia plant of the present invention, comprising a step of detecting whether or not a genotype at a position corresponding to position 37 of SEQ ID NO: 1 is C/C (e.g., the presence and/or the absence of the genetic feature of the present invention) from the genome of a test stevia plant (hereinafter, may be referred to as the "screening method A of the present invention").

The presence of the genetic feature of the present invention can be determined by detecting the absence of an allele wherein the base at a position corresponding to position 37 of SEQ ID NO: 1 is other than C (e.g., T) (e.g., an allele comprising the nucleotide sequence of SEQ ID NO: 15, 16 or 17), and optionally, by detecting the presence of an allele wherein the base at a position corresponding to position 37 of SEQ ID NO: 1 is C (e.g., an allele comprising the nucleotide sequence of SEQ ID NO: 4, 5 or 6).

The absence of the genetic feature of the present invention can be determined by detecting the absence of an allele wherein the base at a position corresponding to position 37 of SEQ ID NO: 1 is C (e.g., an allele comprising the nucleotide sequence of SEQ ID NO: 4, 5 or 6), and/or by detecting the presence of an allele wherein the base at a position corresponding to position 37 of SEQ ID NO: 1 is other than C (e.g., T) (e.g., an allele comprising the nucleotide sequence of SEQ ID NO: 15, 16 or 17).

Specific examples of methods of detecting the genetic features of the present invention include, but not limited to, PCR method, TaqMan PCR method, sequencing method, microarray method, Invader method, TILLING method, RAD method, RFLP method, PCR-SSCP method, AFLP method, SSLP method, CAPS method, dCAPS method, ASO method, ARMS method, DGGE method, CCM method, DOL method, MALDI-TOF/MS method, TDI method, padlock probe method, molecular beacon method, DASH method, UCAN method, ECA method, PINPOINT method, PROBE method, VSET method, Survivor assay, Sniper assay, Luminex assay, GOOD method, LCx method, SNaPshot method, Mass ARRAY method, pyrosequencing method, SNP-IT method, melting curve analysis method, etc.

In one embodiment, the genetic feature of the present invention can be detected by dCAPS method using the following primer set and a restriction enzyme.
- Primer set:
   A primer set comprising a forward primer comprising the sequence of SEQ ID NO: 19 which is positioned at the 3' end, and an optional sequence which is added to the 5' end of the sequence and is of any consecutive upstream bases following position 304 of SEQ ID NO: 13 (e.g., a consecutive sequence of any length), and a reverse primer comprising a sequence (e.g., SEQ ID NO: 8 or 20) complementary to a sequence of any consecutive 20 bases or more which is positioned downstream of position 39 of SEQ ID NO: 9 or 10. The sequences of the primers can be optimized within a range that satisfies the conditions described above. For the optimization of primer design, see, for example, Sambrook and Russell (supra). Each of the primers may be 15 to 50 base long, 18 to 48 base long, 20 to 45 base long, 30 to 65 base long, or the like.
- Restriction enzyme: AflIII

In a specific embodiment, the genetic feature of the present invention can be detected by dCAPS method using the forward primer consisting of the sequence of SEQ ID NO: 7, the reverse primer consisting of the sequence of SEQ ID NO: 8, and the restriction enzyme AflIII.

The screening method A of the present invention may further comprise a step of evaluating the content of a nutrient component and/or the expression level of FRO2 in the test stevia plant tissue for which the genetic features of the present invention have been detected. The evaluation of the content of a nutrient component and the expression level of FRO2 is as described in the section relating to the plant of the present invention. In this embodiment, the screening method A of the present invention may be applied to daughter plants obtained by selecting individuals having a higher content of a nutrient component and/or a lower expression level of FRO2 from among the test stevia plants in which the genetic feature of the present invention is/are detected, and crossing the selected individuals with another stevia plants. Thus, the screening method A of the present invention may comprise one or more of the following steps.
(i) Detecting the genetic feature of the present invention from the genome of a test stevia plant;
(ii) evaluating the content of a nutrient component and the expression level of FRO2 in the test stevia plant tissue in which the genetic feature has been detected;
(iii) selecting an individual having a higher content of a nutrient component and/or a lower expression level of FRO2 from among the test stevia plants in which the genetic feature of the present invention has been detected;
(iv) crossing the selected individual with another stevia plant;
(v) detecting the genetic feature of the present invention from the genome of daughter plants obtained by crossing;
(vi) evaluating the content of a nutrient component and the expression level of FRO2 in the tissue of the daughter plants in which the genetic feature has been detected; and
(vii) selecting individuals having a higher content of a nutrient component and/or a lower expression level of FRO2 from among the daughter plants in which the genetic feature is detected.

Individuals to be selected having higher content of a nutrient component may be, for example, up to 50%, up to 40%, up to 30%, up to 25%, up to 20%, up to 15%, up to 10%, up to 5%, up to 4%, up to 3%, up to 2%, or up to 1% of the test stevia plants in which the genetic feature of the present invention has been detected, with respect to higher content of a nutrient component. Individuals to be selected having lower expression level of FRO2 may be, for example, up to 50%, up to 40%, up to 30%, up to 25%, up to 20%, up to 15%, up to 10%, up to 5%, up to 4%, up to 3%, up to 2%, or up to 1% of the test stevia plants in which the genetic feature of the present invention has been detected, with respect to lower expression level of FRO2. Other stevia plants to be crossed may or may not contain the genetic feature of the present invention. In the above embodiment, steps (iv) to (vii) can be repeated a plurality of times. In this way, stevia plants having a higher content of a nutrient component and/or a lower expression level of FRO2 can be screened.

In the screening method of the present invention, the test stevia plant may be a natural plant, a non-genetically modified plant or a genetically modified plant. Non-genetically modified plants are as described in the section relating to the method of producing the plant of the present invention.

In the screening method of the present invention, the test stevia plant may include a stevia plant subjected to a mutagenesis treatment and a progeny plant thereof. The mutagenesis treatment is as described in the section relating to the plant of the present invention, and includes treatment with a mutagen, treatment with radiation or irradiation with light, and the like.

The present invention also provides the above-mentioned primer set, e.g., the primer set comprising the forward primer comprising the nucleotide sequence of SEQ ID NO: 7 or 19 and the reverse primer comprising the nucleotide sequence of SEQ ID NO: 8 or 20. The present invention further provides a primer set capable of amplifying a region having a nucleotide sequence of SEQ ID NO: 1 by PCR, for example, a primer set with a forward primer comprising a nucleotide sequence of SEQ ID NO: 2 and a reverse primer comprising a nucleotide sequence of SEQ ID NO: 3.

In addition, the present invention provides a probe capable of detecting the presence and/or absence of the genetic features of the present invention, which may be referred to as the "probe of the present invention" hereinafter. The probe of the present invention may have a structure suitable for various detection methods for the presence and/or absence of the genetic feature of the present invention. For example, the probe of the present invention may comprise a nucleotide sequence complementary to a portion of a genome comprising a variation site of the present invention. Non-limiting examples of such probes include those comprising a nucleotide sequence selected from SEQ ID NOs: 4 to 6, and 16 to 18. Of these sequences, SEQ ID NOs: 4 to 6 are specific for the allele comprising the variation of the present invention, and SEQ ID NOs: 16 to 18 are specific for alleles not containing the variation of the present invention. The presence of the genetic feature of the present invention may be detected by detection of an allele comprising the variation of the present invention and/or by non-detection of an allele not comprising the variation of the present invention, and the absence of the genetic feature of the invention by non-detection of an allele comprising the variation of the present invention and/or by detection of an allele not comprising the variation of the present invention. The probes of the present invention preferably have a label. Non-limiting examples of such labels include fluorescent labels, luminescent labels, radioactive labels, dyes, enzymes, quenchers, binding moieties with detectable labels, and the like. In a specific embodiment, the probe of the present invention has a nucleotide sequence complementary to the nucleotide sequence selected from SEQ ID NOs: 4 to 6, and 16 to 18 and a label.

The present invention further provides a kit, for example, a kit for screening, comprising a primer set comprising a forward primer comprising the sequence of SEQ ID NO: 19 which is positioned at the 3' end, and an optional sequence which is added to the 5' end of the sequence and is of any consecutive upstream bases following position 304 of SEQ ID NO: 13 (e.g., a consecutive sequence of any length), and a reverse primer comprising a sequence (e.g., SEQ ID NO: 8 or 20) complementary to a sequence of any consecutive 20 bases or more which is positioned downstream of position 39 of SEQ ID NO: 9 or 10, for example, a primer set comprising the forward primer consisting of the sequence of SEQ ID NO: 7 and the reverse primer consisting of the sequence of SEQ ID NO: 8, and the restriction enzyme AflIII.

The present invention also provides a screening kit comprising a primer set capable of amplifying by PCR a region having a nucleotide sequence selected from the group consisting of SEQ ID NOs: 4 to 6 and 16 to 18, and a probe of the present invention.

These primer sets, probes and kits can be used to detect the genetic feature(s) of the present invention, used in the screening methods of the present invention, and the like. These primer sets and kits may also comprise an instruction including an explanation on the detection of genetic feature of the present invention and on the screening method of the present invention, e.g., a written instruction, information of a site comprising information regarding the method of use (e.g., URL and 2D code), and media, e.g., a flexible disk, a CD, a DVD, a Blu-ray disk, a memory card, a USB memory, etc., having recorded thereon information regarding the method of use.

In one embodiment, the present invention provides a screening kit for the stevia plant of the present invention comprising a reagent for detecting whether or not a genotype at a position corresponding to position 37 of SEQ ID NO: 1 is C/C (e.g., the presence and/or the absence of the genetic features of the present invention). The reagent may comprise a primer and/or a probe for use in CAPS method, dCAPS method or TaqMan PCR method. In a specific embodiment, the reagent for detecting the presence and/or the absence of the genetic features of the present invention comprises a combination of a primer set and a restriction enzyme for detecting the genetic features of the present invention by dCAPS method, a combination of a primer set including, for example, the forward primer comprising the sequence of SEQ ID NO: 7 and the reverse primer comprising the sequence of SEQ ID NO: 8, and the restriction enzyme Af1III, or a combination of a primer set usable in TaqMan PCR method or the like for amplifying a site related to the genetic features of the present invention (e.g., a site comprising a sequence selected from SEQ ID NOS: 1, 4 to 6, and 16 to 18) and a probe having a nucleotide sequence complementary to a site related to the genetic features of the present invention (e.g., a site comprising a sequence selected from SEQ ID NOS: 4 to 6 and 16 to 18).

### 4. Method of producing extract derived from plant and product comprising the extract

In a further aspect, the present invention provides a method of producing an extract containing at least one nutrient component selected from the group consisting of minerals (e.g., iron, zinc, phosphorus, copper, molybdenum, etc.), amino acids (e.g., alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, etc.), RebD, and RebM, comprising a step of obtaining an extract from the plant of the present invention, or a seed or a leaf (e.g., dried leaf or fresh leaf) of the plant (hereinafter, may be referred to as the "extract production method of the present invention"). The present invention further provides a method of producing a nutrient component purified product, comprising a step of purifying at least one of the above-mentioned nutrient component from an extract obtained by the extract production method of the present invention (hereinafter, may be referred to as the "nutrient component purified product production method of the present invention").

Specifically, the present invention provides a method of producing a nutrient component purified product, comprising a step of obtaining an extract containing the above-mentioned nutrient component from the stevia plant of the present invention, the stevia plant screened for by the screening method A of the present invention, or the stevia plant produced by the method of the present invention, and a step of purifying the above-mentioned nutrient component from the obtained extract.

The extract containing the above-mentioned nutrient component can be obtained by reacting a fresh leaf or a dried leaf of the plant of the present invention with a suitable solvent (e.g., an aqueous solvent such as water or an organic solvent such as an alcohol, ether or acetone). For the extraction conditions, etc., of, e.g., RebD or RebM, see a method described in Ohta et al. (supra) or WO2010/038911.

Individual nutrient component, e.g., RebD or RebM, can be purified from the extract containing the above-mentioned nutrient component by use of a method known in the art such as a gradient of ethyl acetate or any of other organic solvents:water, HPLC, gas chromatography, TOF-MS, or UPLC.

In one embodiment, the extract obtained by the extract production method of the present invention (hereinafter, referred to as the "extract of the present invention") contains at least one of the nutrient components described above in a higher content than a control stevia plant having the genotype C/T at a position corresponding to position 37 of SEQ ID NO: 1. The magnitude of the content as compared with that in the control stevia plant is as described above in the section relating to the plant of the present invention.

The extract of the present invention thus obtained and/or the nutrient component purified product (e.g., minerals (e.g., iron, zinc, phosphorus, copper, molybdenum, etc.), amino acids (e.g., alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, etc.), RebD and/or RebM) obtained by the method of producing a nutrient component purified product according to the present invention can be mixed with other component(s) to produce a medicament, flavor or food or beverage containing the nutrient components described above. Accordingly, in an alternative aspect, the present invention provides a method of producing a medicament, a flavor or a food or beverage, comprising a step of mixing the extract of the present invention and/or the nutrient component purified product obtained by the method of producing a nutrient component purified product according to the present invention with other component(s). The present invention further provides a medicament, flavor or food or beverage containing the nutrient components described above, obtained by the production method. In this context, the food or beverage means a beverage and a food. Thus, in a certain embodiment, the present invention provides a medicament, flavor, beverage or food and also provides a method of producing the medicament, the flavor, the beverage or the food.

The present invention also provides a herbal tea product comprising a dry matter of the plant of the present invention. A site of the plant included in the herbal tea product of the present invention is not particularly limited, but may be any site such as a leaf, a stem, or a root, and is preferably a dry matter of a leaf containing a larger amount of a nutrient component. The herbal tea product may be in a form of a ground matter, a powder, or a granule of a dried plant (e.g., a dried leaf), or a tea bag obtained by filling the ground matter, the powder or the granule in a water-permeable bag.

### 5. Nucleotide sequence related to plant of present invention

In another aspect, the present invention provides a nucleotide sequence related to the stevia plant of the present invention. The nucleotide sequence related to a stevia plant of the present invention comprises or consists of a nucleotide sequence selected from SEQ ID NOs: 4 to 6, 21 and 22.

### 6. Method of screening for nutrient component-rich plant

In another aspect, the present invention provides a method of screening for a plant having a high content of a nutrient component, the method comprising a step of measuring an FRO2 expression level (hereinafter, referred to as the "screening method B of the present invention"). According to the present invention, it is found that there is a negative correlation between an FRO2 expression level and a content of a nutrient component, and a plant having a high content of a nutrient component can be screened for with the FRO2 expression level used as an index.

In the screening method B of the present invention, an individual having a low FRO2 expression level can be selected. A low FRO2 expression level may mean that the FRO2 expression level is equal to or lower than a median of the expression level in a test plant population. In another embodiment, a low FRO2 expression level may mean that the FRO2 expression level is included in top 50%, 40%, 30%, 25%, 20%, 15%, 10%, 5%, 4%, 3%, 2% or1% in the test plant population. In a specific embodiment, a low FRO2 expression level may mean that, for example, an FPKM value of FRO2 analyzed through sequencing by NGS is 3.2 or less, 3.1 or less, 3.0 or less, 2.9 or less, 2.8 or less, 2.7 or less, 2.6 or less, 2.5 or less, 2.4 or less, 2.3 or less, 2.2 or less, 2.1 or less, 2.0 or less, 1.9 or less, 1.8 or less, 1.7 or less, 1.6 or less, 1.5 or less, 1.4 or less, 1.3 or less, 1.2 or less, 1.1 or less, 1.0 or less, 0.9 or less, 0.8 or less, 0.7 or less, 0.6 or less, or 0.5 or less.

The FRO2 expression level can be measured by any known method for measuring a gene expression level, such as sequencing by NGS or the like, various hybridization methods utilizing a nucleic acid molecule encoding FRO2 or a nucleic acid molecule specifically hybridizing its unique fragment (e.g., in situ hybridization), Northern blotting, Southern blotting, various PCRs, immunoprecipitation using a substance, such as an antibody, capable of specifically recognizing FRO2, EIA (e.g., ELISA, etc.), RIA (e.g., IRMA, RAST, RIST, etc.), Western blotting, immunohistochemistry, immunocytochemistry, flow cytometry, or MRI. The genomic sequence, CDS sequence, and the amino acid sequence of stevia FRO2 are respectively shown in SEQ ID NOS: 13, 14, and 15. Also, the sequences of FRO2 genes of other plants are known (e.g., Arabidopsis thaliana: NM _001331284 (nucleotide sequence: SEQ ID NO: 23, amino acid sequence: SEQ ID NO: 24), Medicago truncatula: XM_013589350 (nucleotide sequence: SEQ ID NO: 25, amino acid sequence: SEQ ID NO: 26), Helianthus annuus: XM_022180093 (nucleotide sequence: SEQ ID NO: 27, amino acid sequence: SEQ ID NO: 28), and Manihot esculenta: XM_021755804 (nucleotide sequence: SEQ ID NO: 29, amino acid sequence: SEQ ID NO: 30)), and the sequence of FRO2 gene of another plant can be obtained by homology analysis of a sequence obtained by sequencing. For measuring the FRO2 expression level, any plant tissue, such as a leaf, a stem, a root, or a flower, in which FRO2 is probably expressed can be used.

A nutrient component includes, but not limited to, e.g., minerals (e.g., iron, zinc, phosphorus, copper, molybdenum, etc.), amino acids (e.g., alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, etc.), sweet component such as RebD and RebM, and the like.

The screening method B of the present invention may further comprise a step of evaluating the content of a nutrient component in the test plant having a low FRO2 expression level. The evaluation of the content of a nutrient component is as described in the section relating to the plant of the present invention. In this embodiment, the screening method B of the present invention may be applied to daughter plants obtained by selecting individuals having a higher content of a nutrient component from among the test stevia plants having a low FRO2 expression level, and crossing the selected individuals with another plants. Thus, the screening method B of the present invention may comprise one or more of the following steps.
(i) Measuring FRO2 expression level in a test plant;
(ii) evaluating the content of a nutrient component in the tissue of the test stevia having low FRO2 expression level;
(iii) selecting an individual having a higher content of a nutrient component from among the test plants having low FRO2 expression level;
(iv) crossing the selected individual with another plant;
(v) measuring FRO2 expression level in daughter plants obtained by crossing;
(vi) evaluating the content of a nutrient component in the tissue of the daughter plants having low FRO2 expression level; and
(vii) selecting individuals having a higher content of a nutrient component from among the daughter plants having low FRO2 expression level.

Individuals to be selected having higher content of a nutrient component may be, for example, up to 50%, up to 40%, up to 30%, up to 25%, up to 20%, up to 15%, up to 10%, up to 5%, up to 4%, up to 3%, up to 2%, or up to 1% of the test plants in which the genetic feature of the present invention has been detected, with respect to higher content of a nutrient component. Other plants to be crossed may or may not have low FRO2 expression level. In the above embodiment, steps (iv) to (vii) can be repeated a plurality of times. In this way, stevia plants having a higher content of a nutrient component can be screened.

### EXAMPLES

Hereinafter, the present invention will be described with reference to Examples, etc. However, the present invention is not limited by these specific embodiments.

### [Example 1] Relationship between RebD/RebM content and genotype C/C

Individuals derived from commercially available stevia seeds were subjected to selection based on the development and growth condition, the foliar morphology, the contents of RebA, RebD, and RebM, etc. to obtain an individual group I. In the individual group I, genomic DNA was extracted from a fresh leaf of each individual and sequenced with NGS (HiSeq 2500, Illumina, Inc.). Also, a steviol glycoside content in a fresh leaf of each individual was quantitatively determined by LC/MS-MS (Shimadzu LCMS8050). Specifically, 0.25 g of fresh leaves was dried by freeze drying, and 0.05 g of homogenized dry matter thereof was added into pure water. Extraction by ultrasonic treatment for 20 minutes, and centrifugation and filtration were performed to obtain 0.33 mL of a liquid extract. The concentrations of steviol glycosides (RebA, RebB, RebC, RebD, RebE, RebF, RebG, RebM, RebN and stevioside) were quantitatively determined by LC/MS-MS analysis on this liquid extract in a LCMS8050 ion mode (Shimadzu LCMS8050). The average contents (%DW) of RebD, RebM, RebA and TSG in a dried leaf of an individual of each genotype, and ratios (%) of RebD and RebM contents in the TSG content are shown in Table 1, a ratio, obtained when the average content of each steviol glycoside in an individual having the genotype C/T or the ratio of RebD or RebM content in the TSG content is defined as 1, of a corresponding numerical value in the individual having the genotype C/C is shown in Table 2, and distributions of the RebD and RebM contents (%DW) in a dried leaf of the individual of each genotype are shown in FIG. 2. As indicated by these results, a large number of individuals having the genotype C/C were included in individuals having high RebD and RebM contents. For example, individuals having a total content of RebD and RebM in a dried leaf of 1.3% by weight or more all had the genotype C/C, and 75% of individuals having the genotype C/C had a total content of RebD and RebM in a dried leaf of 1.3% by weight or more. On the contrary, among individuals having the genotype C/T, no individual had a total content of RebD and RebM in a dried leaf of 1.3% by weight or more. Also, the RebA content and the TSG content tended to be lower in the individuals having the genotype C/C than in the individuals having the genotype C/T. Note that the TSG content means a total content of RebA, RebB, RebC, RebD, RebE, RebF, RebG, RebM, RebN and stevioside, and RebDM means the sum of RebD and RebM.

**Table 1 Average contents of steviol glycosides in dried leaves of individuals having respective genotypes**

| | RebD (%DW) | RebM (%DW) | RebDM (%DW) | RebA (%DW) | TSG (%DW) | RebD/TSG (%) | RebM/TSG (%) | RebDM/TSG (%) |
|---|---|---|---|---|---|---|---|---|
| C/C | 1.26 (0.19-2.63) | 0.43 (0.08-1.19) | 1.69 (0.44-3.35) | 3.43 (1.24-9.89) | 7.19 (3.03-15.70) | 18.78 (4.71-29.18) | 6.62 (0.59-14.49) | 25.40 (5.7-34.19) |
| C/T | 0.44 (0.10-0.96) | 0.26 (0.02-0.60) | 0.70 (0.13-1.20) | 7.07 (3.16-11.38) | 11.38 (5.13-19.09) | 4.93 (0.65-13.90) | 3.21 (0.15-7.58) | 8.13 (0.86-21.47) |

| | | | | | | | | |
|---|---|---|---|---|---|---|---|---|
| * A parenthesized range indicates a range from the minimum value to the maximum value. | | | | | | | | |

**Table 2 Ratios of average contents of steviol glycosides in individuals having the genotype C/C**

| RebD | RebM | RebDM | RebA | TSG | RebD/TSG | RebM/TSG | RebDM/TSG |
|---|---|---|---|---|---|---|---|
| 2.85 | 1.67 | 2.41 | 0.48 | 0.63 | 3.81 | 2.06 | 3.12 |

### [Example 2] Relationship between FRO2 expression level and genotype C/C

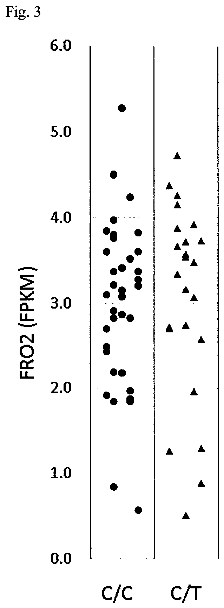

An SNP related to the genotype C/C is positioned in FRO2 gene. A total RNA was extracted from a fresh leaf of each individual of the individual group I and sequenced with NGS (HiSeq 2500, Illumina, Inc.). Based on the thus obtained data, an FPKM value of FRO2 gene in each individual was calculated. As a result, a large number of individuals having the genotype C/C were included in individuals having a low FRO2 gene expression level (FIG. 3). For example, about 69% of individuals having an FRO2 gene expression level (FPKM value) in a dried leaf of 2.5 or less had the genotype C/C, and the FRO2 gene expression level (FPKM value) in a dried leaf was 2.5 or less in 31% of individuals having the genotype C/C. On the contrary, among individuals having the genotype C/T, about 21% of individuals had an FRO2 gene expression level (FPKM value) in a dried leaf of 2.5 or less. These results suggest that the FRO2 gene expression level tends to be lower in individuals having the genotype C/C than in individuals having the genotype C/T.

### [Example 3] Relationship between mineral content and genotype C/C

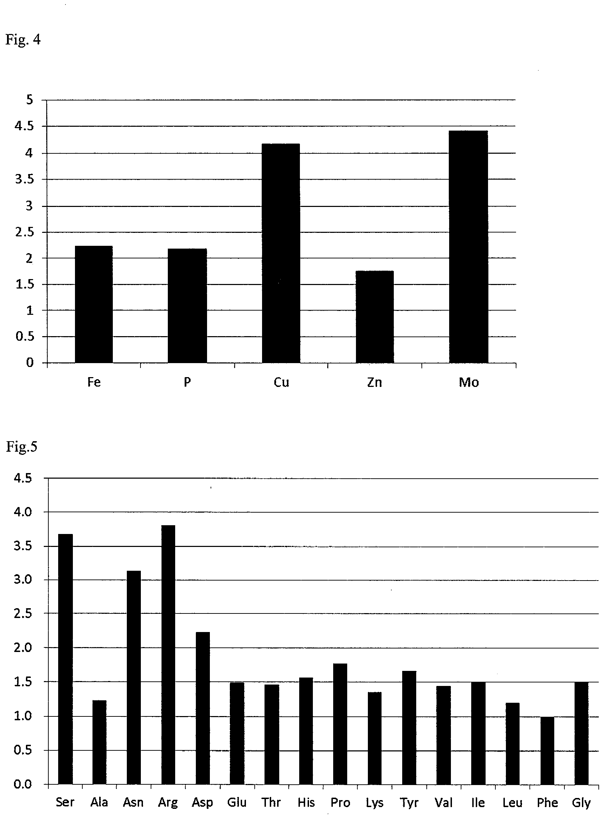

In representative 2 individuals of each genotype belonging to the individual group I, mineral components in an extract from a dried leaf were quantitatively determined. Fresh leaves were collected from each individual, and thermally dried at 65°C for about 17 hours. 1 mL of hot water (of 95°C or more) was added per 100 mg of the thus obtained dried leaves, the resultant was mixed with a stirrer for 5 minutes, and nitric acid was added thereto in an amount of 10 µL per mL of the solution, followed by standing overnight. The thus obtained sample was subjected to ICP-MS (Agilent 7500cx, Agilent Technologies) to measure a concentration of each element (with attached MassHunter Workstation software). An average content of each mineral component in a dried leaf of the individual of each genotype is shown in Table 3, and a ratio of the average content of each mineral component in the individual having the genotype C/C obtained when the average content of each mineral component in the individual having the genotype C/T is defined as 1 is shown in Table 4 and FIG. 4. The contents of all the mineral components were higher in the individual having the genotype C/C than in the individual having the genotype C/T.

**Table 3 Average contents (ppb) of each mineral component in dried leaves of individuals having respective genotypes**

| | Fe | P | Cu | Zn | Mo |
|---|---|---|---|---|---|
| C/C | 6.32 (6.11-6.53) | 28225.99 (23132.37-33319.61) | 131.12 (123.81-138.44) | 94.03 (90.73-97.33) | 1.28 (0.76-1.80) |
| C/T | 2.84 (2.80-2.87) | 12988.64 (12914.16-13063.13) | 31.49 (28.15-34.83) | 53.80 (52.85-54.74) | 0.29 (0.27-0.31) |

| | | | | | |
|---|---|---|---|---|---|
| * A parenthesized range indicates a range from the minimum value to the maximum value. | | | | | |

**Table 4 Ratios of average contents of each mineral component in individuals having the genotype C/C**

| Fe | P | Cu | Zn | Mo |
|---|---|---|---|---|
| 2.2 | 2.2 | 4.2 | 1.7 | 4.4 |

### [Example 4] Relationship between amino acid content and genotype C/C

In representative 2 individuals of each genotype belonging to the individual group I, free amino acids in an extract from a dried leaf were quantitatively determined. Fresh leaves were collected from each individual, and thermally dried at 65°C for about 17 hours. 5 mL of hot water (of 95°C or more) was added per 100 mg of the thus obtained dried leaves, the resultant was mixed with a stirrer for 5 minutes, subjected to a sonication for 5 minutes, then centrifuged for 10 minutes at 12000 rpm, and the supernatant was collected. The thus obtained supernatant sample was subjected to HPLC (Chromaster 5210/5260, Hitachi High-Tech) to measure a concentration of each amino acid (using Empower 3, Waters). An average content of each amino acid in a dried leaf of the individual of each genotype is shown in Tables 5 and 6, and a ratio of the average content of each amino acid in the individual having the genotype C/C obtained when the average content of each mineral component in the individual having the genotype C/T is defined as 1 is shown in Table 7 and FIG. 5. The contents of all the amino acids were higher in the individual having the genotype C/C than in the individual having the genotype C/T.

**Table 5 Average contents (ppm) of each amino acid in dried leaves of individuals having respective genotypes 1**

| | Ser | Ala | Asn | Arg | Asp | Glu | Thr | His |
|---|---|---|---|---|---|---|---|---|
| C/C | 96.63 (43.5-149.8) | 25.23 (21.9-28.5) | 7.45 (4.1-10.8) | 2.92 (1.9-3.9) | 0.85 (0.4-1.3) | 2.08 (1.5-2.7) | 5.35 (5.3-5.4) | 1.28 (1.2-1.4) |
| C/T | 26.27 (5.7-46.9) | 20.42 (14.4-26.4) | 2.38 (1.4-3.3) | 0.71 (0.0-1.5) | 0.38 (0.0-0.8) | 1.40 (0.0-2.8) | 3.66 (2.8-4.5) | 0.82 (0.5-1.2) |

| | | | | | | | | |
|---|---|---|---|---|---|---|---|---|
| * A parenthesized range indicates a range from the minimum value to the maximum value. | | | | | | | | |

**Table 6 Average contents (ppm) of each amino acid in dried leaves of individuals having respective genotypes 2**

| | Pro | Lys | Tyr | Val | Ile | Leu | Phe | Gly |
|---|---|---|---|---|---|---|---|---|
| C/C | 4.84 (4.0-5.7) | 3.76 (3.1-4.4) | 3.47 (3.1-3.8) | 5.30 (5.1-5.7) | 4.48 (3.8-5.1) | 8.97 (7.4-10.6) | 11.19 (7.2-15.1) | 0.39 (0.0-0.8) |
| C/T | 2.73 (2.2-3.3) | 2.78 (1.7-3.8) | 2.08 (1.4-2.7) | 3.65 (2.5-4.8) | 2.97 (1.8-4.2) | 7.50 (5.4-9.6) | 11.25 (5.0-17.5) | 0.26 (0.0-0.5) |

| | | | | | | | | |
|---|---|---|---|---|---|---|---|---|
| * A parenthesized range indicates a range from the minimum value to the maximum value. | | | | | | | | |

**Table 7 Ratios of average contents of each amino acid in individuals having the genotype C/C**

| Ser | Ala | Asn | Arg | Asp | Glu | Thr | His | Pro | Lys | Tyr | Val | Ile | Leu | Phe | Gly |
|---|---|---|---|---|---|---|---|---|---|---|---|---|---|---|---|
| 3.7 | 1.2 | 3.1 | 3.8 | 2.2 | 1.5 | 1.5 | 1.6 | 1.8 | 1.4 | 1.7 | 1.5 | 1.5 | 1.2 | 1.0 | 1.5 |

### [Example 5] Relationship between RebD/RebM content and FRO2 expression level

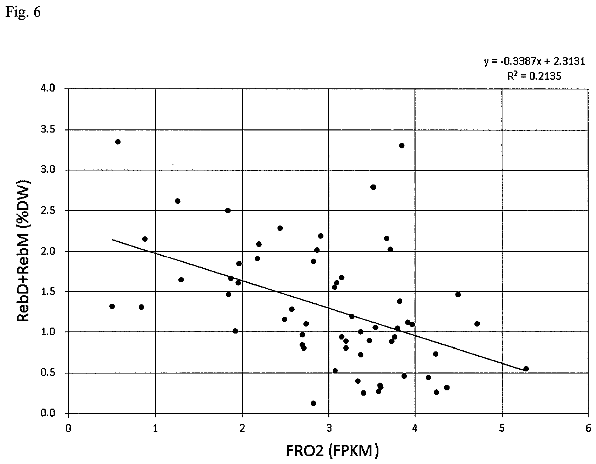

When the total contents of RebD and RebM and the FRO2 expression levels of the respective individuals of the individual group I obtained in Examples 1 and 2 were plotted in a scatter plot, there was a negative correlation therebetween (FIG. 6). Also, average values of the RebD and RebM contents in individual groups having FRO2 expression levels falling in top/bottom 50%, 25%, 15% and 10% are shown in Table 8.

**Table 8 Relation between FRO2 expression level and RebD/RebM content**

| FRO2 expression level | RebD (%DW) | RebM (%DW) | RebDM (%DW) |
|---|---|---|---|
| Top 50% | 0.71 | 0.30 | 1.01 |
| Bottom 50% | 1.15 | 0.43 | 1.58 |
| Top 25% | 0.69 | 0.32 | 1.01 |
| Bottom 25% | 1.41 | 0.51 | 1.92 |
| Top 15% | 0.49 | 0.29 | 0.79 |
| Bottom 15% | 1.41 | 0.60 | 2.00 |
| Top 10% | 0.49 | 0.24 | 0.74 |
| Bottom 10% | 1.42 | 0.64 | 2.07 |

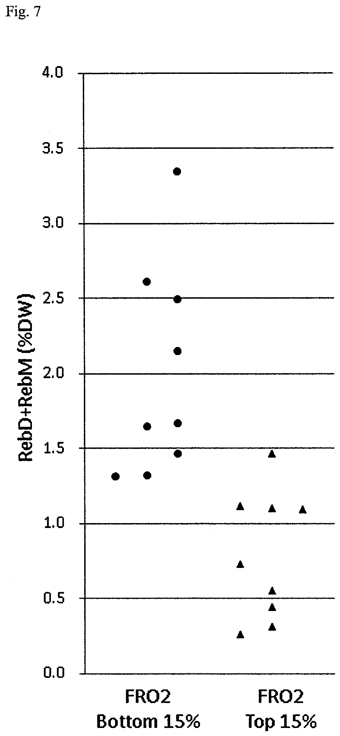

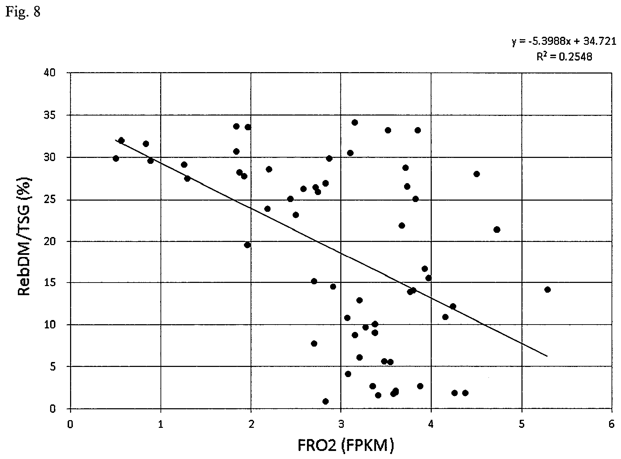

As indicated by these results, the averages of the RebD and RebM contents were significantly lower in the group of individuals having a low FRO2 expression level than in the group having a high FRO2 expression level. Comparison results of the total content of RebD and RebM between the individual group having an FRO2 expression level falling in bottom 15% and the individual group having an FRO2 expression level falling in top 15% are shown in FIG. 7. Also, it was revealed that there is a stronger correlation between a ratio of the total content of RebD and RebM to the TSG content (RebDM/TSG) and the FRO2 expression level (FIG. 8).

## Claims

1. A stevia plant having the genotype C/C at a position corresponding to position 37 of SEQ ID NO: 1.

2. The plant according to claim 1, wherein the plant contains at least one nutrient component selected from the group consisting of iron, zinc, phosphorus, copper, molybdenum, amino acid, rebaudioside D, and rebaudioside M in a larger amount than a control stevia plant having a genotype C/T at a position corresponding to position 37 of SEQ ID NO: 1, and/or has an FRO2 expression level lower than in the control stevia plant.

3. The plant according to claim 1 or 2, wherein the plant is a non-genetically modified plant.

4. A seed, a tissue, a tissue culture or a cell of the plant according to any one of claims 1 to 3.

5. The tissue, tissue culture or cell according to claim 4, which is selected from an embryo, a meristem cell, a pollen, a leaf, a root, a root apex, a petal, a protoplast, a leaf section and a callus.

6. A method of producing a stevia plant containing at least one component selected from the group consisting of iron, zinc, phosphorus, copper, molybdenum, amino acid, rebaudioside D, and rebaudioside M in a larger amount than in a control stevia plant having a genotype C/T at a position corresponding to position 37 of SEQ ID NO: 1, and/or having an FRO2 expression level lower than in the control stevia plant, the method comprising a step of crossing the plant according to any one of claims 1 to 3 with a second stevia plant.

7. The method according to claim 6, wherein the second plant is the plant according to any one of claims 1 to 3.

8. An extract of the plant according to any one of claims 1 to 3, or of the seed, tissue, tissue culture or cell according to claim 4 or 5, wherein the extract comprises at least one nutrient component selected from iron, zinc, phosphorus, copper, molybdenum, amino acid, rebaudioside D, and rebaudioside M.

9. A method of producing an extract comprising at least one nutrient component selected from iron, zinc, phosphorus, copper, molybdenum, amino acid, rebaudioside D, and rebaudioside M, wherein the method comprises a step of obtaining an extract from the plant according to any one of claims 1 to 3, or from the seed, tissue, tissue culture or cell according to claim 4 or 5.

10. A method of producing a food or beverage, a sweetener composition, a flavor or a medicament, comprising
a step of providing an extract of the plant according to any one of claims 1 to 3, an extract of the seed, tissue, tissue culture or cell according to claim 4 or 5, or the extract according to claim 8, and
a step of adding the extract to a raw material for the food or beverage, sweetener composition, flavor or medicament.

11. A herbal tea product comprising a dry matter of the plant according to any one of claims 1 to 3.

12. A method of screening for the stevia plant according to any one of claims 1 to 3, the method comprising a step of detecting, in a test stevia plant, whether or not a genotype at a position corresponding to position 37 of SEQ ID NO: 1 is C/C.

13. The method according to claim 12, wherein the step of detecting a genetic feature is performed by use of sequencing, dCAPS method or TaqMan PCR method.

14. The method according to claim 12 or 13, further comprising a step of evaluating the content of at least one nutrient component selected from iron, zinc, phosphorus, copper, molybdenum, amino acid, rebaudioside D, and rebaudioside M in a test stevia plant tissue.

15. A screening kit for the stevia plant according to any one of claims 1-3, comprising a reagent for detecting whether or not a genotype at a position corresponding to position 37 of SEQ ID NO: 1 is C/C.

16. The kit according to claim 15, wherein the reagent comprises a primer and/or a probe for use in dCAPS method or TaqMan PCR method.

17. A method of screening for a plant having a high content of at least one nutrient component selected from the group consisting of iron, zinc, phosphorus, copper, molybdenum, amino acid, rebaudioside D, and rebaudioside M, the method comprising a step of measuring an FRO2 expression level.
